(19) Europäisches Patentamt
European Patent Office
Office européen des brevets

(11) Veröffentlichungsnummer : **0 023 887**
**B1**

(12) # EUROPÄISCHE PATENTSCHRIFT

(45) Veröffentlichungstag der Patentschrift : **07.11.84**

(21) Anmeldenummer : **80810233.9**

(22) Anmeldetag : **28.07.80**

(51) Int. Cl.³ : **C 07 D515/04**, C 07 D205/08// C07C149/18, C07C143/68, C07C153/017, C07F7/18, C07F9/65, C07D499/00, A61K31/43 ,(C07D515/04, 291/00, 205/00)

(54) **3-Substituierte bicyclische Azetidinonderivate, Verfahren zu ihrer Herstellung und Zwischenprodukte, sowie deren Herstellung.**

(30) Priorität : **01.08.79 CH 7077/79**
**01.07.80 CH 5083/80**

(43) Veröffentlichungstag der Anmeldung : **11.02.81 Patentblatt 81/06**

(45) Bekanntmachung des Hinweises auf die Patenterteilung : **07.11.84 Patentblatt 84/45**

(84) Benannte Vertragsstaaten :
**BE CH DE FR GB IT LI NL SE**

(56) Entgegenhaltungen :
**DE-A- 2 205 123**
**DE-A- 2 724 560**
**JOURNAL OF THE CHEMICAL SOCIETY, Perkin Transactions I, 12, 1974, D.H.R. BARTON et al. "Transformations of penicillins. Part VII. Vinylic sulphoxides from penicillins and their reactions",Seiten 1459-1468**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Pfaendler, Hans-Rudolf, Dr.**
**Carl Spittelerstrasse 2**
**CH-4410 Liestal (CH)**

**EP 0 023 887 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung ist ein neues Verfahren zur Herstellung von 3-substituierten-4-trans-Hydroxyalkylthio- oder Hydroxyalkylsulfonyl-azetidinonen, Zwischenprodukte, welche für die Herstellung dieser Verbindungen entwickelt worden sind, sowie Verfahren zur Herstellung dieser Zwischenprodukte.

In der DE-A-2 724 560 sind 3-substituierte-4-Hydroxyäthyl-azetidinone beschrieben, welche als Zwischenprodukte zur Herstellung von Carbapenemen verwendet werden. Diese Zwischenprodukte führen zu cis-, trans-Gemischen von Carbapenemen.

In der am 19.9.1979 veröffentlichten Europäischen Patentanmeldung Nr. 3960 sind 3-substituierte-4-Hydroxyalkylthio- oder -Hydroxyalkylsulfonylazetidinone der Formel :

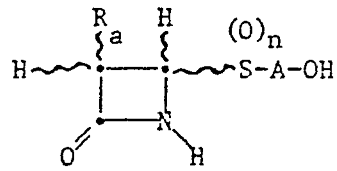

(V)

beschrieben, worin $R_a$ einen über ein Kohlenstoffatom mit dem Ringkohlenstoffatom verbundenen, gesättigten oder ungesättigten, gegebenenfalls substituierten, aliphatischen, cycloaliphatischen, cycloaliphatischaliphatischen, aromatischen oder araliphatischen Kohlenwasserstoffrest mit bis zu 18, bevorzugt bis zu 10, Kohlenstoffatomen oder einen gegebenenfalls substituierten Heterocyclyl- oder Heterocyclylniederalkylrest mit bis zu 10 Kohlenstoffatomen und 1 bis 4 Heteroatomen aus der Gruppe Stickstoff, Sauerstoff und/oder Schwefel darstellt, und worin die Substituenten von diesen Resten $R_a$ Hydroxy, Niederalkoxy, Niederalkanoyloxy, in Salzform vorliegendes Hydroxysulfonyloxy, Halogen, Mercapto, Niederalkylmercapto, Carboxyl, Niederalkoxycarbonyl, Carbamoyl, Cyan, Nitro, in Salzform vorliegendes Sulfo, oder gegebenenfalls durch Niederalkyl oder Acyl mono- oder di-substituiertes oder durch Niederalkylen disubstituiertes Amino sind, A einen Niederalkenylenrest mit 2-3 Kohlenstoffatomen zwischen dem Schwefel und Sauerstoff und n 0 oder 2 bedeuten.

Diese Verbindungen lassen sich als Zwischenprodukte zur Herstellung von Penemen verwenden. Das in der Europäischen Patentanmeldung Nr. 3960 für Verbindungen der Formel V beschriebene Herstellungsverfahren führt zu cis-, trans-Gemischen.

Der vorliegenden Erfindung liegt die Aufgabe zugrunde, ausgehend von leicht zugänglichen Ausgangsmaterialien Azetitidinone der Formel V herzustellen, worin der Substitutent $R_a$ in 3-Stellung und die Hydroxyalkylthio- bzw. Hydroxyalkylsulfonylgruppe in 4-Stellung die trans-Anordnung einnehmen. Eine Trennung von cis-, trans-Isomeren ist dann nicht notwendig.

Diese Aufgabe wird durch das erfindungsgemässe Verfahren gelöst, welches dadurch gekennzeichnet ist, dass man ein Acyloxyazetidinon der Formel

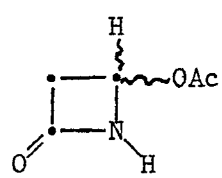

(IV)

worin Ac einen Acylrest darstellt, mit einem Hydroxyniederalkylmercaptan der Formel HS—A—OH in Gegenwart einer geeigneten Base umsetzt und gewünschtenfalls in einer erhältlichen Verbindung die Gruppe —S—A—OH in eine andere Gruppe —S—A—OH überführt und eine erhältliche Verbindung der Formel

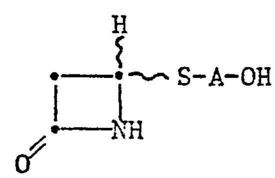

(II)

worin A die unter Formel V genannte Bedeutung hat, mit einer Carbonylverbindung der Formel

$$R_b—(C{=}O)—R_c \quad (III)$$

worin jeder der Reste $R_b$ und $R_c$ Wasserstoff oder einen organischen Rest darstellt, der mit einem

Kohlenstoffatom mit der Carbonylgruppe verknüpft ist, wobei $R_b$ und $R_c$ untereinander verknüpft sein können, oder einem Derivat davon, in Gegenwart eines sauren Reagenzes umsetzt und in einer erhältlichen Verbindung der Formel

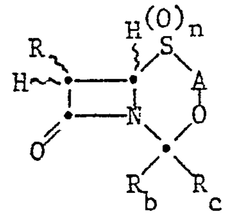

(I)

worin R Wasserstoff bedeutet, A die unter Formel V, $R_b$ und $R_c$ die unter Formel III genannten Bedeutungen haben und n 0 bedeutet, einen Rest $R_a$ einführt und gewünschtenfalls eine erhältliche Verbindung in eine Verbindung der Formel I überführt, worin n für 2 steht und/oder ein verfahrensgemäss erhältliches Isomerengemisch in die einzelnen Isomere auftrennt und die Verbindung der Formel I in Gegenwart eines geeigneten Solvolysereagenz solvolysiert und gewünschtenfalls eine erhältliche Verbindung der Formel V, worin n für 0 steht, in eine Verbindung der Formel V überführt, worin n für 2 steht, und/oder eine erhältliche Verbindung in die einzelnen Isomere auftrennt.

Die weiter vorn und im folgenden verwendeten allgemeinen Definitionen haben im Rahmen der Beschreibung der vorliegenden Erfindung vorzugsweise die folgenden Bedeutungen :

$R_a$ ist insbesondere gegebenenfalls substituiertes Niederalkyl oder Niederalkenyl, gegebenenfalls funktionell abgewandeltes Carboxy, oder gegebenenfalls substituiertes Cycloalkyl, Cycloalkenyl, Cycloalkylniederalkyl, Cycloalkylniederalkenyl, Cycloalkenylniederalkyl, Phenyl, Phenylniederalkyl, oder Phenylniederalkenyl. Substituenten von solchen Resten sind z. B. gegebenenfalls funktionell abgewandelte, wie gegebenenfalls verätherte oder veresterte Hydroxy- oder Mercaptogruppen, z. B. Hydroxy, Niederalkoxy, z. B. Methoxy oder Aethoxy, Niederalkanoyloxy, z. B. Acetyloxy oder Propionyloxy, in Salzform vorliegendes Hydroxysulfonyloxy, Halogen, z. B. Chlor oder Brom, oder Niederalkylmercapto, z. B. Methylthio, gegebenenfalls funktionell abgewandelte Carboxylgruppen, wie Carboxyl, Niederalkoxycarbonyl, z. B. Methoxycarbonyl oder Aethoxycarbonyl, Carbamoyl oder Cyan, ferner Nitro, in Salzform vorliegendes Sulfo, oder gegebenenfalls substituiertes, wie durch Niederalkyl, z. B. Methyl oder Aethyl, Acyl, wie Niederalkanoyl, z. B. Acetyl, mono- oder disubstituiertes, oder durch Niederalkylen, z. B. 1,4-Butylen oder 1,5-Pentylen, disubstituiertes Amino.

Ein Niederalkylrest $R_a$ enthält bis zu 7, insbesondere bis zu 4 Kohlenstoffatome, und ist z. B. Methyl, Aethyl, Propyl, Isopropyl, Butyl, Isobutyl, tert.-Butyl oder Pentyl. Substituiertes Niederalkyl $R_a$ ist in erster Linie substituiertes Methyl, Aethyl oder Propyl, wobei Substituenten insbesondere in 1-, aber auch in 2- oder in 3-Stellung stehen, und ist beispielsweise Hydroxyniederalkyl, wie Hydroxymethyl, Hydroxyäthyl oder Hydroxypropyl, Niederalkoxyniederalkyl, wie Niederalkoxymethyl, Niederalkoxyäthyl oder Niederalkoxypropyl, z. B. Methoxymethyl, Methoxyäthyl oder Methoxypropyl, Niederalkanoyloxyniederalkyl, wie Niederalkanoyloxymethyl, Niederalkanoyloxyäthyl oder Niederalkanoyloxypropyl, z. B. Acetyloxymethyl, Propionyloxymethyl, Acetyloxyäthyl, Acetyloxypropyl, in Salzform, z. B. als Alkalimetall-, wie Natriumsalz, oder als Ammoniumsalz vorliegendes Hydroxysulfonyloxyniederalkyl, wie Hydroxysulfonyloxymethyl, Hydroxysulfonyloxyäthyl oder Hydroxysulfonyloxypropyl, Halogenniederalkyl, wie Halogenmethyl, Halogenäthyl oder Halogenpropyl, z. B. Chlor- oder Bromäthyl, oder Chlor- oder Brompropyl, Niederalkylthioniederalkyl, wie Methylthiomethyl, Methylthioäthyl, Methylthiopropyl oder tert.-Butylthiomethyl, Niederalkoxycarbonylniederalkyl, wie Niederalkoxycarbonylmethyl oder Niederalkoxycarbonyläthyl, z. B. Methoxycarbonylmethyl, Methoxycarbonyläthyl, Aethoxycarbonylmethyl oder Aethoxycarbonyläthyl, Cyanniederalkyl, wie Cyanmethyl oder Cyanäthyl, Sulfoniederalkyl, wie Sulfomethyl, Sulfoäthyl oder Sulfopropyl, worin die Sulfogruppe in Salzform, z. B. als Alkalimetall-, wie Natriumsalz oder auch als Ammoniumsalz vorliegt, oder gegebenenfalls geschütztes, z. B. auch acetyliertes, Aminoniederalkyl, wie Aminomethyl, Aminoäthyl oder Aminopropyl.

Ein Niederalkenylrest $R_a$ enthält 2 bis 7, insbesondere 2 bis 4 Kohlenstoffatome, und ist z. B. Vinyl, Allyl oder 2- oder 3-Butenyl. Substituiertes Niederalkenyl kann die gleichen Substituenten tragen wie substituiertes Niederalkyl.

Eine gegebenenfalls funktionell abgewandelte Carboxylgruppe $R_a$ ist eine freie oder eine veresterte oder amidierte Carboxylgruppe, wie Niederalkoxycarbonyl, z. B. Methoxy-, Aethoxy- oder tert.-Butoxycarbonyl, Arylniederalkoxycarbonyl, wie Benzyloxy-, p-Nitrobenzyloxy- oder Diphenylmethoxycarbonyl, Aryloxycarbonyl, wie gegebenenfalls, z. B. durch Halogen, wie Chlor, Niederalkoxy, wie Methoxy oder Nitro, substituiertes Phenyloxycarbonyl, wie Phenyloxycarbonyl, o-, m- oder p-Chlorphenyloxy-, Pentachlorphenyloxy-, o-, m- oder p-Methoxyphenyloxy- oder p-Nitrophenyloxycarbonyl, Aminocarbonyl oder substituiertes, wie durch Niederalkyl, z. B. Methyl oder Aethyl, mono- oder disubstituiertes Aminocarbonyl.

Ein Cycloalkylrest $R_a$ weist z. B. 3 bis 7 Kohlenstoffatome auf und ist z. B. Cyclopropyl, Cyclobutyl, Cyclopentyl oder Cyclohexyl, während ein Cycloalkylniederalkylrest $R_a$ beispielsweise 4 bis 7 Kohlenstoffatome enthält und z. B. Cyclopropylmethyl, Cyclobutylmethyl, Cyclopentylmethyl oder Cyclohexylmethyl darstellt.

Ein Cycloalkenylrest $R_a$ ist ein entsprechender Cycloalkylrest mit ein oder gegebenenfalls zwei C-C-Doppelbindungen, wie Cyclohexenyl, z. B. 1-Cyclohexenyl, oder Cyclohexadienyl, z. B. 1,4-Cyclohexadienyl.

Ein Cycloalkylniederalkenylrest oder Cycloalkenylniederalkylrest $R_a$ ist z. B. Cyclohexylvinyl, Cyclohexylallyl, bzw. Cyclohexenylmethyl oder 1,4-Cyclohexadienylmethyl.

Ein Phenyl- oder ein Phenylniederalkyl-, z. B. Benzyl- oder 1- oder 2-Phenyläthylrest $R_a$ ist, vorzugsweise im aromatischen Rest, z. B. durch Niederalkyl, wie Methyl oder Aethyl, Niederalkoxy, wie Methoxy, oder Halogen, wie Fluor oder Chlor, ferner durch Nitro oder Amino gegebenenfalls substituiert, wobei Phenylniederalkyl in α-Stellung z. B. durch Hydroxy, Hydroxysulfonyloxy, Carboxy, Sulfo oder Amino substituiert sein kann.

In einem Heterocyclyl- oder Heterocyclylniederalkylrest $R_a$ ist Heterocyclyl ein über ein Kohlenstoffatom gebundener Rest vorzugsweise aromatischen Charakters, wie Pyridyl, z. B. 2-, 3- oder 4-Pyridyl, Thienyl, z. B. 2-Thienyl, oder Furyl, z. B. 2-Furyl, oder ein entsprechender Pyridyl-, Thienyl- oder Furylniederalkyl-, insbesondere -methylrest, wobei Heterocyclylniederalkyl in α-Stellung z. B. durch Hydroxy, Hydroxysulfonyloxy, Carboxy, Sulfo oder Amino substituiert sein kann.

Ein Phenyl- oder Heterocyclylniederalkenylrest $R_a$ ist ein wie ein entsprechender Niederalkylrest substituierten Niederalkenylrest, z. B. Phenylvinyl oder Furylallyl.

Ein Niederalkylenrest A mit 2-3 Kohlenstoffatomen zwischen dem Schwefel und Sauerstoff ist in erster Linie Aethylen und 1,2-Propylen, kann jedoch auch 1,3-Propylen, 1,2- 2,3- oder 1,3-Butylen sein.

Die Erfindung betrifft insbesondere ein Verfahren zur Herstellung von Verbindungen der Formel V, worin $R_a$ Niederalkyl, Niederalkenyl, Hydroxyniederalkyl, Niederalkoxyniederalkyl, Niederalkanoyloxyniederalkyl, gegebenenfalls, z. B. durch Niederalkyl, Niederalkoxy, Halogen und/oder Nitro, substituiertes Arylniederalkoxycarbonyloxyniederalkyl, oder in Salzform vorliegendes Hydroxysulfonyloxyniederalkyl ist, A Aethylen oder 1,2-Propylen darstellt, und n 0 oder 2 bedeutet, die Stereoisomere von Verbindungen der Formel V und Mischungen von diesen Stereoisomeren.

Die Erfindung betrifft in erster Linie ein Verfahren zur Herstellung von Verbindungen der Formel V, worin $R_a$ Niederalkyl mit bis zu 4 Kohlenstoffatomen, z. B. Methyl, Aethyl, Propyl, oder Butyl, Hydroxyniederalkyl, insbesondere 1-Hydroxyniederalkyl, mit bis zu 4 Kohlenstoffatomen, z. B. Hydroxymethyl, 1, Hydroxyäthyl oder 1-Hydroxypropyl, Niederalkenyl mit bis zu 4 Kohlenstoffatomen, Niederalkoxyniederalkyl, insbesondere 1-Niederalkoxyniederalkyl, worin Niederalkyl und Niederalkoxy je bis zu 4 Kohlenstoffatome enthält, z. B. Methoxymethyl, 1-Methoxyäthyl oder 1-Methoxypropyl, Niederalkanoyloxyniederalkyl, insbesondere 1-Niederalkanoyloxyniederalkyl, worin Niederalkanoyloxy und Niederalkyl je bis zu 4 Kohlenstoffatome enthalten, z. B. Acetoxymethyl, Proprionyloxymethyl oder 1-Acetyloxyäthyl, gegebenenfalls, z. B. durch Halogen und/oder Nitro substituiertes Phenylniederalkoxycarbonyloxyniederalkyl, insbesondere entsprechendes 1-Phenylniederalkoxycarbonyloxyniederalkyl, oder in Salzform vorliegendes Hydroxysulfonyloxyniederalkyl, worin Niederalkyl bis zu 4 Kohlenstoffatome enthält, insbesondere 1-Hydroxysulfonyloxyniederalkyl, z. B. Hydroxysulfonyloxymethyl, 1-Hydroxysulfonyloxyäthyl oder 1-Hydroxysulfonyloxypropyl, bedeutet und A Aethylen oder 1,2-Propylen darstellt und worin n 0 oder 2 sein kann, die Stereoisomere von Verbindungen der Formel V und Mischungen von diesen Stereoisomeren.

Die Erfindung betrifft vor allem Verbindungen der Formel V, worin R Wasserstoff oder den Rest $R_a$ bedeutet, wobei $R_a$ für Niederalkyl mit bis zu 4-Kohlenstoffatomen, insbesondere Methyl, Niederalkenyl mit bis zu 4 Kohlenstoffatomen, 1-Hydroxyniederalkyl mit bis zu 4 Kohlenstoffatomen, insbesondere 1-Hydroxyäthyl steht, worin die 1-Hydroxygruppe gegebenenfalls durch eine Acylgruppe, wie durch den Acylrest einer organischen Carbonsäure oder Sulfonsäure, wie gegebenenfalls substituiertes Niederalkanoyl, z. B. Acetyl oder Trifluoracetyl, oder gegebenenfalls durch Halogen und/oder Nitro substituiertes Phenylniederalkoxycarbonyl, z. B. p-Nitrobenzyloxycarbonyl geschützt sein kann, A Aethylen oder 1,2-Propylen ist, und worin n 0 oder 2 ist, die Stereoisomere von Verbindungen der Formel V und Mischungen von diesen Stereoisomeren.

In einem Ausgangsmaterial der Formel IV is Ac der Acylrest einer organischen Carbon- oder Sulfonsäure, insbesondere gegebenenfalls substituiertes Niederalkanoyl, z. B. Acetyl, Trifluoracetyl oder Formyl, oder gegebenenfalls substituiertes Benzoyl, z. B. Benzoyl, Ausgangsmaterialien der Formel IV und Verfahren zu ihrer Herstellung sind bekannt.

Ein Hydroxyniederalkylmercaptan enthält 2-3 Kohlenstoffatome zwischen den beiden Heteroatomen und ist z. B. 3-Mercaptopropanol, vor allem 2-Mercaptoäthanol oder 2-Mercapto-1-propanol.

Geeignete Basen sind starke organische oder anorganische Basen, z. B. 1,5-Diazabicyclo[5.5.0]undec-5-en, Alkalimetall- oder Erdalkalimetallhydroxide, vor allem Natriumhydroxid. Letztere Basen werden bevorzugt in konzentrierten wässrigen Lösungen zugefügt.

Als Lösungsmittel eignen sich schwach polare Lösungsmittel, z. B. Tetrahydrofuran oder Dioxan, oder stärker polare Lösungsmittel, z. B. Dimethylformamid, Dimethylsulfoxid, oder Alkohole, z. B. Methanol oder Aethanol. Bevorzugt findet die Umsetzung in Gegenwart von Wasser bei Temperaturen von etwa − 20° bis etwa 100°, vorzugsweise − 10° bis Zimmertemperatur, statt.

Bei der Umsetzung eines Acyloxyazetidinons der Formel IV mit einem Hydroxyniederalkylmercaptan

wird ein racemisches (4R,S)-Gemisch erhalten. Setzt man bei dieser Reaktion eine Verbindung HS-A-OH ein, worin A ein Chiralitätszentrum enthält, z. B. gegebenenfalls an der Hydroxygruppe geschütztes (2R)-2-Mercapto-1-propanol, so erhält man ein Diasteromerengemisch von Verbindungen der Formel II.

Das so erhältliche Diastereomerengemisch lässt sich gewünschtenfalls wie weiter unten beschrieben in die Antipoden auftrennen.

Verbindungen der Formel II, die in A ein weiteres Chiralitätszentrum besitzen, können Diastereomerengemische bilden. Solche Diastereomerengemische sind beispielsweise durch Umsetzung eines (4R,S)-Acyloxyazetidinons der Formel IV mit einem Antipoden eines 2-Mercapto-1-propanols, z. B. (2R)-2-Mercapto-1-propanol erhältlich.

Solch ein Diastereomerengemisch lässt sich gewünschtenfalls mit üblichen Methoden in die einzelnen Antipoden auftrennen, die sich gemäss der obigen Umsetzung ohne Konfigurationsveränderung zu Antipoden der Formel I weiterverarbeiten lassen.

Verbindungen der Formel II und das oben erähnte (2R)-2-Mercapto-1-propanol sind neu und bilden ihrerseits zusammen mit ihrer Herstellung einen weiteren Gegenstand der vorliegenden Erfindung.

(2R)-2-Mercapto-1-propanol ist bei der Umsetzung mit einem Acyloxyazetidinon der Formel IV an der Hydroxylgruppe durch in der Cephalosporin- oder Penicillinchemie übliche Hydroxylschutzgruppen gegebenenfalls geschützt. Als Hydroxylschutzgruppen kommen vor allem Silylgruppen, z. B. Trimethylsilyl- oder Acylgruppen, z. B. Acetylgruppen in Frage.

Die Herstellung von (2R)-2-Mercapto-1-propanol erfolgt, indem man die Hydroxygruppe eines (S)-Milchsäureesters in Gegenwart einer organischen Base, z. B. Triäthylamin, durch einen Säurerest einer organischen Säure, z. B. den Mesylrest, verestert. Durch Umsetzung der erhaltenen Verbindung mit einem geeigneten Thiosalz, z. B. Kaliumthioacetat, wird die Konfiguration des an der Hydroxygruppe veresterten Milchsäureesters umgekehrt und die veresterte Hydroxygruppe durch eine substituierte Thiogruppe ausgetauscht. Anschliessende Reduktion mit einem geeigneten Reduktionsmittel, z. B. Lithiumaluminiumhydrid, ergibt das (2R)-2-Mercapto-1-propanol.

In einem Ausgangsmaterial der Formel III sind organische Reste in erster Linie Niederalkyl, z. B. Methyl, Aethyl, n-Propyl oder Isopropyl, gegebenenfalls substituiertes Phenyl, oder Phenylniederalkyl, z. B. Benzyl, oder, wenn zusammengenommen, Niederalkylen vorzugsweise mit 4-6 Kohlenstoffatomen, z. B. 1,4-Butylen oder 1,5-Pentylen.

Carbonylverbindungen der Formel III sind vorzugsweise Ketone, worin jeder der Reste $R_b$ und $R_c$ einen einwertigen organischen Rest, in erster Linie Niederalkyl mit bis zu 4 Kohlenstoffatomen, z. B. Methyl oder Aethyl, Aryl oder Arylniederalkyl mit bis zu 10 Kohlenstoffatomen, z. B. Phenyl oder Benzyl darstellt, insbesondere Niederalkanone, z. B. Aceton, oder, wenn $R_b$ und $R_c$ zusammengenommen einen zweiwertigen organischen Rest bedeuten, zyklische Ketone, insbesondere Cycloalkanone, z. B. Cyclohexanon, ebenfalls optisch aktive Ketone oder Aldehyde, z. B. Kampfer.

Derivate der Carbonylverbindung der Formel III sind z. B. Acetale oder Enoläther, vor allem Acetondimethylketal.

Saure Reagenzien sind starke und schwache Säuren des Brönstedt Typs, beispielsweise Mineralsäuren, z. B. Schwefelsäure oder Salzsäure, organische Säuren, z. B. Paratoluolsulfonsäure, oder Lewis-Säuren, z. B. Bortrifluorid-Aetherat, Kupfersulfat oder Eisen(III)-chlorid.

Das bei der Reaktion einer Verbindung der Formel II mit einer Carbonylverbindung der Formel III sich bildende Wasser kann aus dem Reaktionsgemisch z. B. durch azeotrope Destillation in üblicher Weise mit Hilfe eines Wasserabscheiders entfernt werden.

Dazu eignen sich als Lösungsmittel insbesondere aromatische Kohlenwasserstoffe, z. B. Benzol oder Toluol.

Die Reaktion wird vorzugsweise bei erhöhten Temperaturen wie von etwa 40° bis etwa 150°, vor allem bei der Siedetemperatur des jeweiligen Reaktionsgemisches durchgeführt.

In die Reaktion können sowohl Antipoden von Verbindungen der Formel II, als auch deren racemische oder diastereomere Mischungen eingesetzt werden. Bei der Reaktion bleibt die Konfiguration der Chiralitätszentren unverändert.

Verbindungen der Formel I sind neu und ebenfalls Gegenstand der vorliegenden Erfindung.

In eine Verbindung der Formel I kann man einen organischen Rest $R_a$ z. B. durch Behandeln einer verfahrensgemäss erhältlichen Verbindung der Formel I, worin R für Wasserstoff steht, mit einem geeigneten Metallierungsreagens, gefolgt von einer dem organischen Rest $R_a$ entsprechenden reaktionsfähigen Verbindung, in eine Verbindung der Formel I, worin R Wasserstoff bedeutet, einführen.

Geeignete Metallierungsreagentien sind substituierte und unsubstituierte Alkalimetallamide, Alkalimetallhydride oder Alkalimetallniederalkylverbindungen, worin das Alkalimetall Natrium oder insbesondere Lithium ist, z. B. Natrium- oder Lithiumamid, Lithium-bis-trimethylsilylamid, Natriumhydrid, Lithiumhydrid und bevorzugt Lithiumdiisopropylamid und Butyllithium.

Eine dem organischen Rest $R_a$ entsprechende reaktionsfähige Verbindung ist beispielsweise eine Verbindung der Formel $R_a$—X, worin X eine nukleofuge Abgangsgruppe, beispielsweise ein Halogenatom, z. B. Chor, Brom oder Jod, oder eine Sulfonyloxygruppe, z. B. Mesyloxy oder Tosyloxy darstellt, oder eine dem Rest $R_a$ entsprechende Carbonylverbindung, in erster Linie ein Keton oder eine Aldehydverbindung der Formel $R_a'$—(C=O)—$R_a''$, worin $R_a'$ und $R_a''$ unabhängig voneinander Wasserstoff oder eine Gruppe, die zusammen mit der aus der Carbonylgruppe hervorgehenden Carbinolgruppe einen

Rest $R_a$ bildet, bedeutet. $R_a'$ und $R_a''$ sind unabhängig voneinander bevorzugt Wasserstoff oder Niederalkyl.

Für die Metallierungsreaktion geeignete Lösungsmittel dürfen keinen aktiven Wasserstoff enthalten und sind z. B. Kohlenwasserstoffe, z. B. Hexan, Benzol, Toluol oder Xylol, schwach polare Aether, z. B. Diäthyläther, Tetrahydrofuran oder Dioxan, oder Säureamide, z. B. Hexamethylphosphorsäuretriamid.

Das metallierte Zwischenprodukt braucht nicht isoliert, sondern kann im Anschluss an die Metallierungsreaktion mit einer dem organischen Rest $R_a$ entsprechenden reaktionsfähigen Verbindung umgesetzt werden. Die Metallierungsreaktion erfolgt bei Temperaturen von etwa $-100°$ bis etwa $+100°$, vorzugsweise unterhalb $-30°$. Die weitere Umsetzung kann bei der gleichen Temperatur und gegebenenfalls langsamen Erwärmen bis zu 100° erfolgen.

Für die Metallierungsreaktion können sowohl die Antipoden von Verbindungen der Formel I als auch deren racemische oder diastereomere Mischungen eingesetzt werden.

Der Angriff der dem organischen Rest $R_a$ entsprechenden reaktionsfähigen Verbindung an das Substrat erfolgt im allgemeinen stereospezifisch. Wird als Ausgangsmaterial ein (4S)-Azetidinon der Formel I benutzt, erhält man überwiegend ein (3R,4S)-4-$R_a$-Azetidinon. Wird ein (4R)-Azetidinon eingesetzt, erhält man überwiegend ein (3S,4R)-Azetidinon der Formel I. Es ergibt sich also überwiegend eine trans- Verbindung.

Bei der Reaktion mit einem Keton oder Aldehyd der Formel $R_a'$—(C=O)—$R_a''$ wird in 1-Stellung der Seitenkette ein weiteres Chiralitätszentrum gebildet, das üblicherweise die (R,S)-Konfiguration besitzt. Dabei ist je nach der Stellung der Hydroxygruppe zwischen einer Threo-trans- und einer Erythro-trans-Verbindung zu unterscheiden.

Ein diastereomeres Gemisch, bestehend aus einer Threo-trans- und Erythro-trans-Verbindung, lässt sich mit den üblichen Methoden, z. B. durch fraktionierte Kristallisation, chromatographisch oder dergleichen, in die einzelnen Antipoden auftrennen.

In einer verfahrensgemäss erhältlichen Verbindung der Formel I, worin $R_a$ einen in 1-Stellung durch Hydroxy substituierten organischen Rest $R_a$, z. B. 1-Hydroxy-alkyl, darstellt, kann man eine Hydroxygruppe in an sich bekannter Weise, z. B. durch Veräthern oder Verestern, schützen bzw. substituieren.

Geeignete Schutzgruppen sind Acylreste einer organischen Carbon- oder Sulfonsäure, insbesondere gegebenenfalls substituiertes Niederalkanoyl, z. B. Acetyl, Trifluoracetyl, gegebenenfalls substituiertes Benzyl oder Phenylniederalkanoyl, z. B. Benzoyl, gegebenenfalls substituiertes Niederalkoxycarbonyl oder Phenylniederalkoxycarbonyl, z. B. p-Nitrobenzyloxycarbonyl, ferner 2-Oxacycloalkyl, z. B. 2-Tetrahydropyranyl, oder gegebenenfalls substituierte Silyl- oder Stannylgruppen, z. B. Trimethylsilyl. Eine Hydroxyschutzgruppe kann in an sich bekannter Weise, z. B. durch Behandeln mit reaktionsfähigen Derivaten, wie Anhydriden, z. B. Säurehalogeniden oder Ketenen, Halogeniden, oder entsprechenden ungesättigten Verbindungen, eingeführt werden.

Die Ueberführung von Verbindungen der Formel I, worin der Index n für 0 steht, in Verbindungen der Formel I, worin n für 2 steht, erfolgt durch Oxydation, und zwar durch Behandlung mit Sulfid- in Sulfongruppen überführenden Mitteln, insbesondere mit Wasserstoffperoxid, organischen Persäuren, insbesondere aliphatischen Percarbonsäuren, z. B. Peressigsäure, Perbenzoesäure, Chlorperbenzoesäure, z. B. m-Chlorperbenzoesäure, oder Monoperphthalsäure, mit oxidierenden anorganischen Säuren oder deren Salzen, z. B. Salpetersäure, Chromsäure, Kaliumpermanganat, oder einem Alkalimetallhypochlorit, z. B. Natriumhypochlorit, sowie auch durch anodische Oxidation. Die Oxidation wird bevorzugt in einem geeigneten inerten Lösungsmittel, beispielsweise einem Halogenkohlenwasserstoff, z. B. Methylenchlorid, Chloroform oder Tetrachlorkohlenstoff, einem Alkohol, z. B Methanol oder Aethanol, einem Keton, z. B. Aceton, einem Aether, z. B. Diäthyläther, Dioxan oder Tetrahydrofuran, einem Amid, z. B. Dimethylformamid, einem Sulfon, z. B. Dimethylsulfon, einer flüssigen organischen Carbonsäure, z. B. Essigsäure, oder in Wasser oder einem Gemisch dieser Lösungsmittel, insbesondere einem wasserhaltigen Gemisch, z. B. wässriger Essigsäure, bei Raumtemperatur, oder unter Kühlen oder leichtem Erwärmen, d. h. bei etwa $-20$ bis etwa $+90°$, bevorzugt bei etwa $+18$ bis etwa $+30°$, durchgeführt. Die Oxidation kann auch stufenweise durchgeführt werden, indem zunächst bei niederer Temperatur, d. h. bei etwa $-20$ bis etwa 0° bis zur Sulfoxidstufe oxidiert wird, das gegebenenfalls isoliert wird, worauf in einem zweiten Schritt, bevorzugt bei höherer Temperatur, etwa bei Raumtemperatur, das Sulfoxid zum Sulfon, d. h. dem 1,1-Dioxid der Formel (I), oxidiert wird.

Zur Aufarbeitung kann gegebenenfalls noch vorhandenes überschüssiges Oxidationsmittel durch Reduktion, insbesondere durch Behandeln mit einem Reduktionsmittel, wie einem Thiosulfat, z. B. Natriumthiosulfat, zerstört werden.

Die Solvolyse einer Verbindung der Formel I zu Verbindungen der Formel V erfolgt mit geeigneten Solvolysereagenzien.

Diese sind beispielsweise organische Säuren, z. B. Niederalkancarbonsäuren, z. B. Eisessig oder Ameisensäure, Anhydride von Niederalkancarbonsäuren, z. B. Essigsäureanhydrid, oder Sulfonsäuren, z. B. p-Toluolsulfonsäuren, Mineralsäuren, z. B. Schwefel- oder Salzsäure, Niederalkanole, z. B. Methanol oder Aethanol, oder Niederalkandiole, z. B. Aethylenglykol.

Die genannten Solvolysereagenzien werden unverdünnt oder mit Wasser verdünnt zugegeben. Die Solvolyse kann auch mit reinem Wasser durchgeführt werden.

Die Solvolyse mit dem sauren Reagens findet bevorzugt in wässriger Lösung dieses Reagenses und

bei Temperaturen von etwa − 20° bis etwa 150°, bevorzugt bei Zimmertemperatur bis 110°, statt.

Es lässt sich das Sulfid (n = 0) oder das Sulfon (n = 2) der Formel I solvolysieren. Wird das Sulfid solvolysiert, erfolgt anschliessend die Oxydation zum Sulfon in an sich bekannter Weise, z. B. wie oben beschrieben.

Zur Trennung von erfindungsgemäss erhältlichen Diastereomerengemischen in die Antipoden eignen sich physikalisch-chemische Methoden, in erster Linie die fraktionierte Kristallisation. Brauchbar sind aber auch chromatographische Methoden, vor allem festflüssig-Chromatographie. Leichtflüchtige Diastereomerengemische können auch durch Destillation oder Gaschromatographie getrennt werden.

Eine Methode zur Auftrennung von Racematen besteht in der Chromatographie an optisch aktiven Absorptionsschichten, beispielsweise an Rohrzucker, oder es können die Racemate in optisch aktiven Lösungsmitteln gelöst und der schwerer lösliche optische Antipode auskristallisiert werden, oder man benützt die verschiedene Reaktionsfähigkeit der optischen Antipoden gegenüber biologischem Material wie Mikroorganismen oder isolierten Enzymen, oder man löst die Racemate und kristallisiert einen der optischen Antipoden durch Animpfen mit einer kleinen Menge eines nach den obigen Methoden erhaltenen optisch aktiven Produkte aus.

Die Verbindungen der Formel V können, z. B. wie in nachfolgendem Reaktionsschema gezeigt und in den Beispielen illustriert, in 6-substituierte 2-Penem-3-carbonsäure-Verbindungen übergeführt werden.

2-Penem-3-carbonsäure-Verbindungen, die in 6-Stellung einen Substituenten enthalten, der von einer Acylaminogruppe verschieden ist, und die sowohl gegen Penicillin-empfindliche als auch gegen Penicillin resistente Keime wirksam sind, sind, in der am 19.9.1979 veröffentlichten Europäischen Patentanmeldung Nr. 3960 beschrieben.

Im folgenden Reaktionsschema sind die in der Europäischen Patentanmeldung Nr. 3960 beschriebenen Reaktionsstufen wiedergegeben, die zu 6-substituierten-2-Penem-3-carbonsäuren, siehe Formel VI, führen.

Die Gruppe $—SO_2—A—OH$ in einer Verbindung der Formel V stellt eine nukleofuge Abgangsgruppe dar und wird im folgenden W genannt.

Reaktionsschema

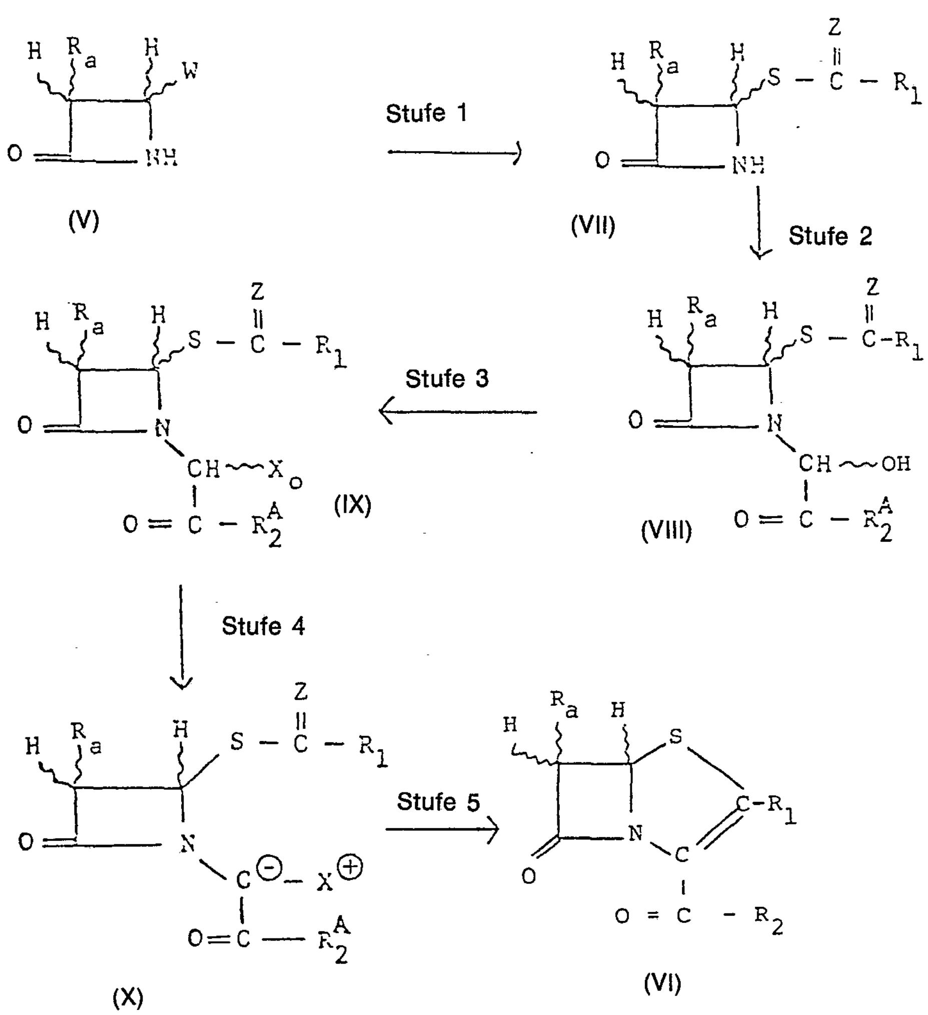


(V) (VII) (IX) (VIII) (X) (VI)

In den Verbindungen der Formeln VII, VIII, IX und X im Reaktionsschema ist Z Sauerstoff, Schwefel oder auch, insbesondere wenn $R_1$ Wasserstoff ist, eine gegebenenfalls durch einen oder zwei Substituenten Y substituierte Methylidengruppe, die durch Oxidation in eine Oxogruppe Z übergeführt werden kann. Ein Substituent Y dieser Methylidengruppe, ist ein organischer Rest, beispielsweise einer der unter $R_1$ erwähnten organischen Reste, wie einer der genannten gegebenenfalls substituierten Niederalkyl-, Cycloalkyl-, Cycloalkylniederalkyl, Phenyl- oder Phenylniederalkylreste, und insbesondere eine der funktionell abgewandelten, wie veresterten, inklusive mit einem optisch aktiven Alkohol, wie 1-Menthol, veresterten Carboxylgruppen. Diese Methylidengruppe trägt bevorzugt einen der genannten Substituenten. Hervorzuheben ist die 2-Carbo-1-methyloxymethylidengruppe Z. Letztere kann zur Herstellung optisch aktiver Verbindungen der Formeln VII, VIII, IX und X verwendet werden.

In einer Verbindung der Formel IX ist $X_0$ eine reaktionsfähige veresterte Hydroxygruppe, insbesondere Halogen oder organisches Sulfonyloxy. In einer Verbindung der Formel X bedeutet $X^{\oplus}$ eine der bei Wittig-Kondensationsreaktion gebräuchlichen Phosphonio- oder Phophonogruppen, insbesondere eine Triaryl-, z. B. Triphenyl-, oder Triniederalkyl-, z. B. Tributylphosphoniogruppe, oder eine durch Niederalkyl, z. B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetall, z. B. Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z. B. Natriumion.

Die aus Verbindungen der Formel V herstellbaren 6-substituierten-2-Penem-3-carbonsäureverbindungen sind insbesondere diejenigen der Formel

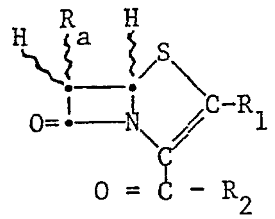

(VI)

worin $R_a$ für einen über ein Kohlenstoffatom mit dem Ringkohlenstoffatom verbundenen organischen Rest steht, $R_1$ für Wasserstoff, einen über ein Kohlenstoffatom mit dem Ringkohlenstoffatom verbundenen organischen Rest oder eine verätherte Mercaptogruppe steht, und $R_2$ Hydroxy oder einen zusammen mit der Carbonylgruppierung —C(=O)— eine geschützte Carboxylgruppe bildenden Rest $R_2^A$ bedeutet, sowie Salze von solchen Verbindungen mit salzbildenden Gruppen.

2-Penem-Verbindungen können wertvolle pharmakologische Eigenschaften aufweisen. So haben Verbindungen der Formel VI, worin $R_a$ und $R_1$ die oben gegebenen Bedeutungen haben, und $R_2$ Hydroxy oder eine zusammen mit der Carbonylgruppe eine, vorzugsweise unter physiologischen Bedingungen leicht spaltbare, veresterte Carboxylgruppe bildende verätherte Hydroxygruppe $R_2^A$ bedeutet, oder pharmakologisch verwendbaren Salze von solchen Verbindungen mit salzbildenden Gruppen antibakterielle Wirkungen. Sie hemmen beispielsweise das Wachstum von gram-positiven und gram-negativen Keimen, wie *Staphylococcus aureus* und *Penicillin-resistentem Staphylococcus aureus, Escherichia coli, Proteus vulgaris, Pseudomonas aeruginosa* und *Pseudomonas aeruginosa R.* Im Disc-Plattentest werden mit erfindungsgemässen Verbindungen der Formel I bei den genannten Keimen mit einer 0,5 %igen Lösung auf Filterpapier (6 mm Durchmesser) Hemmzonen von etwa 12 bis 33 mm Durchmesser festgestellt.

Gleichzeitig analog getestetes Penicillin V verursacht bei Penicillin-empfindlichen *Staphylococcus aureus* Keimein Hemmzonen von 29 bis 33 mm Durchmesser und bei Penicillin-resistenten Keimen von höchstens 9 bis 12 mm. Gegen *Pseudomonas aeruginosa* ist weder Penicillin V noch Penicillin G wirksam.

Die antibakterielle Wirkung *in vitro* kann auch im Agar-Verdünnungs-Test (nach Ericsson) festgestellt werden, wobei gegen grampositive and gramnegative Kokken MIC-Werte von 0,06 bis 8 mcg/ml, und gegen gramnegative Stäbchenbakterien, wie Enterobakterien, Pseudomonas und Haemophilus, von 2 bis 128 mcg/ml ermittelt werden.

*In vivo,* bei der systemischen Infektion an der Maus durch *Streptococcus pyogenes Aronson,* ergeben sich bei subcutaner Application erfindungsgemässer Verbindungen $ED_{50}$-Werte von etwa $\leq$ 1 bis etwa 50 mg/kg.

Hervorzuheben ist insbesondere die Wirksamkeit gegen *Pseudomonas aeruginosa.*

Die Verbindungen hemmen β-Lactamasen und wirken synergistisch im Kombination mit anderen β-Lactamantibiotika.

Diese Verbindungen oder ihre pharmakologisch verwendbaren Salze, können allein oder in Kombination mit anderen Antimicrobica z. B. in Form von antibiotisch wirksamen Präparaten, zur Behandlung von entsprechenden System- oder Organinfektionen, ferner als Futtermittelzusätze, zur Konservierung von Nahrungsmitteln oder als Desinfektionsmittel Verwendung finden.

Die folgenden Beispiele dienen zur Illustration der Erfindung ; Temperaturen sind in Celsiusgraden angegeben. Folgende Abkürzung wird benutzt : DC = Dünnschichtchromatogramm auf Silicagel.

Beispiel 1

4-(2-Hydroxyäthylthio)-2-oxo-azetidin.

Eine Lösung von 12,9 g (0,1 Mol) 4-Acetoxyazetidin-2-on (hergestellt gemäss K. Clauss et al., Lieb. Ann. Chem., *1974,* 539 ; racemisch, Smp. 34°) in 75 ml 95 % Aethanol wird bei – 10° unter Stickstoffatmosphäre unter Rühren mit 10 ml (0,142 Mol) 2-Mercaptoäthanol versetzt und bei – 10° innerhalb 30 Minuten tropfenweise mit 55 ml 2.0 N wässriger Natriumhydroxidlösung versetzt und 60 Minuten lang bei 0° gerührt. Das Gemisch wird mit 2 ml Trifluoressigsäure neutralisiert und im Vakuum-Rotationsverdampfer zur Trockne eingedampft. Der Rückstand wird im Hochvakuum getrocknet, mit 100 g Natriumsulfat versetzt und zweimal mit je 200 ml Methylenchlorid, dann zweimal mit je 200 ml Chloroform extrahiert. Die vereinigten Extrakte werden filtriert und das Filtrat eingedampft. Der viskose Rückstand wird ohne Reinigung weiter verwendet. DC : $R_f$ = 0.12 (Aethylacetat) ; IR-Spektrum ($CH_2Cl_2$) : Absorptionsbanden bei 3 580, 3 380, 1 770 $cm^{-1}$.

Beispiel 2

1. (4R)- und (4S)-4-((2R)-1-Hydroxyprop-2-yl-thio)-2-azetidinon.

Zu einem Gemisch von 2,58 g (20 mMol) 4-Acetoxyazetidin-2-on und 2,76 g (30 mMol) (ER)-2-Mercapto-1-propanol in 12 ml trockenem Tetrahydrofuran wird unter Rühren bei – 20° 30 Minuten lang eine Lösung von 3,6 ml Diazabicyclo[5.4.0]undec-5-en in 6 ml Tetrahydrofuran tropfenweise gegeben. Das Reaktionsgemisch wird während weiterer 30 Minuten bei – 20° gerührt und bei – 40° mit 0,5 ml Trifluoressigsäure versetzt. Dann wird bei 11 mm und 25° im Rotationsverdampfer eineengt und der Rückstand auf 150 g Merck Kieselgel mit EtOAc-Toluol 2 : 1 chromatographiert (20 Fraktionen, je 100 ml) und ein 1 : 1 Gemisch der beiden diastereomeren Titelverbindungen erhalten.

2. Das Ausgangsmaterial (2R)-2-Mercapto-1-propanol wird folgendermassen hergestellt :

2.1. (2S)-2-Methylsulfonyloxypropionsäureäthylester.

Zu einem Gemisch von 23,6 g (22,9 ml, 0.2 Mol) (S)-Milchsäureäthylester und 21,8 ml (0.3 Mol) Triäthylamin in 400 ml trockenem Aether wird unter stetigem Rühren bei Raumtemperatur 15,5 ml Methansulfonylchlorid zugetropft, so dass die Temperatur nicht über 30° steigt. Das Reaktionsgemisch wird dann weitere 60 Minuten bei 25° gerührt. Der entstandene Niederschlag wird durch Filtration entfernt und das Filtrat nacheinander mit 250 ml wässriger 1 N HCl und 250 ml Wasser gewaschen. Die organische Phase wird über Natriumsulfat getrocknet, das Gemisch filtriert und das Lösungsmittel im Rotationsverdampfer entfernt. Der Rückstand von 32,4 g wird im Hochvakuum bei 0,05 mm destilliert, Sdp. 75 °C/0,05 mm. $R_f$-Wert : 0.60 (EtOAc). NMR-Spektrum ($CDCl_3$) in ppm : 5.2, 1H, q, J = 7.5 Hz ; 4.35, 2H, q, J = 8 Hz ; 3.2, 3H, s ; 1.7, 3H, d, J = 7.5 Hz ; 1.35, 3H, t, J = 8 Hz. $[\alpha]_D^{20}$ : – 65° (unverdünnt).

2.2. (2R)-2-Acetylthiopropionsäureäthylester.

In einem Sulfierkolben von 1 000 ml Inhalt wird zu einer Lösung von 19,6 g (2S)-2-Methylsulfonyloxypropionsäureäthylester in 200 ml trockenem tert.-Butanol unter mechanischem Rühren bei Raumtemperatur 15 Minuten lang 17,1 g festes Kaliumthioacetat eingetragen. Das Reaktionsgemisch wird noch 15 Minuten am Rückfluss erhitzt. Nach Abkühlen des Reaktionsgemisches wird mit 1 000 ml Aether versetzt und zweimal mit je 600 ml Wasser gewaschen. Die organische Phase wird über Natriumsulfat getrocknet und nach Filtration des Lösungsmittel im Rotationsverdampfer entfernt. Der Rückstand wird bei 11 mm destilliert. Das Produkt hat einen Siedepunkt von 90°-95° bei 11 mm. $R_f$-Wert 0.63 (EtOAc). IR-Spektrum in $CH_2Cl_2$, $cm^{-1}$ : 2 970, 1 732, 1 695, 1 180. NMR-Spektrum in $CDCl_3$ in ppm : 4.2, 3H, m ; 2.3, 3H, s ; 1.45, 3H, d, J = 7.5 Hz ; 1.25, 3H, t, J = 7 Hz. $[\alpha]_D^{20}$ – 48° (unverdünnt).

2.3. (2R)-2-Mercapto-1-propanol.

In einem Sulfierkolben von 750 ml Inhalt mit mechanischem Rührer wird zu einem Gemisch von 12,16 g Lithiumaluminiumhydrid in 160 ml trockenem Tetrahydrofuran bei 75° Badtemperatur (Rückfluss) innerhalb 30 Minuten eine Lösung von 14,1 g Acetylthiopropionsäureäthylester in 40 ml trockenem Tetrahydrofuran zugetropft und das Reaktionsgemisch während weiterer 60 Minuten am Rückfluss erhitzt. Nach Abkühlen des Gemisches wird tropfenweise bei 0° Badtemperatur 160 ml 2 N wässrige HCl unter ständigem Rühren zugegeben, so dass die Innentemperatur nicht über 30° steigt. Nach 1-stündigem Rühren bei Zimmertemperatur wird das Gemisch durch ein feines Glasfilter filtriert und der

Rückstand mit 700 ml Tetrahydrofuran gewaschen. Die vereinigten Filtrate werden dann im Rotationsverdampfer bei 25° und 11 mm eingeengt. Der Rückstand wird dann mit 500 ml Methylenchlorid versetzt und mit 200 g Natriumsulfat getrocknet. Filtration und erneutes Eindampfen des Filtrates bei 25° und 11 mm ergibt einen flüssigen Rückstand. Er wird bei 11 mm destilliert. Es wird reines Produkt mit einem Siedepunkt von 56° bei 11 mm erhalten. $R_f$-Wert : 0.15 ($CH_2Cl_2$). IR-Spektrum in $CH_2Cl_2$, $cm^{-1}$ : 3 570, 2 920, 2 860, 1 450, 1 390, 1 055, 1 025. NMR-Spektrum in $CDCl_3$ in ppm : 3.3-3.9, 2H, m ; 3.3, 1H, m ; 2.2, 1H, breites s ; 1.45, 1H, d, J = 7.5 Hz ; 1.3, 3H, d, J = 7 Hz. $[\alpha]_D^{20}$ : – 10.4° (unverdünnt). $[\alpha]_D^{20}$ : – 25° ($CHCl_3$, c = 12).

Beispiel 3

1. (4R)- und (4S)-4-((2R)-1-Hydroxyprop-2-yl-thio)-2-azetidinon.

Zu einem Gemisch von 258 mg (2 mMol) 4-Acetoxyazetidin-2-on und 319 mg (2.4 mMol) 1-Acetoxy-(2R)-2-mercaptopropan in 1,2 ml trockenem Tetrahydrofuran wird unter Rühren bei – 30° eine Lösung von 243 mg (1.6 mMol) Diazabicyclo[5.4.0]undec-5-en in 0,8 ml Tetrahydrofuran 15 Minuten lang gegeben. Das Gemisch wird dann während 30 Minuten bei 0° gerührt. Zugabe von 25 ml EtOAc, Waschen mit 25 ml 1 N wässriger HCl und mit 25 ml gesättigter wässriger $NaHCO_3$-Lösung, Trocknen der organischen Phase, Filtration und Eindampfen des Lösungsmittels im Vakuum ergibt ein Gemisch der diastereomeren Acetate. Es wird in 20 ml Methanol aufgelöst und mit 1,5 ml einer 10 %igen, wässrigen $K_2CO_3$-Lösung versetzt und bei Zimmertemperatur während 150 Minuten gerührt, dann mit 120 μl Trifluoressigsäure versetzt und im Vakuum-Rotationsverdampfer eingedampft. Der Rückstand wird im Hochvakuum bei Raumtemperatur getrocknet und anschliessend auf 12 g Merck-Kieselgel mit Toluol-EtOAc 1 : 2 (20 Fraktionen, je 8 ml) chromatographiert. Es wird ein Gemisch beider Titelverbindungen erhalten.

2. Das Ausgangsmaterial 1-Acetoxy-(2R)-2-mercaptopropan kann folgendermassen erhalten werden.

Ein Gemisch von 920 mg des gemäss Beispiel 2, unter 2.3. hergestellten (2R)-2-Mercapto-1-propanols und 1,12 g Essigsäureanhydrid wird während 30 Minuten bei 130° Badtemperatur am Rückfluss erhitzt. Einengen im Vakuum bei 11 mm und 25° ergibt 1,4 g flüssigen Rückstand. Er wird auf 70 g Merck Kieselgel mit Methylenchlorid chromatographiert (15 Fraktionen, je 50 ml). $R_f$-Wert : 0.5 (Toluol-EtOAc 2 : 1). IR-Spektrum in $CH_2Cl_2$, $cm^{-1}$ : 2 950, 1 735, 1 230. NMR-Spektrum in $CDCl_3$ in ppm : 4.1, 2H, d, J = 7 Hz ; 3.2, 1H, m ; 2.1, 3H, s ; 1.65, 1H, d, J = 7 Hz ; 1.35, 3H, d, J = 7 Hz.

Beispiel 4

1. (4R)- und (4S)-4-((2R)-1-Hydroxyprop-2-yl-thio)-2-azetidinon.

Zu einem Gemisch von 258 mg (2 mMol) 4-Acetoxyazetidin-2-on und 401 mg (2.4 mMol) 1-Trimethylsilyloxy-(2R)-2-mercaptopropan in 1,2 ml trockenem Tetrahydrofuran wird unter Rühren bei – 30° eine Lösung von 304 mg (2 mMol) Diazabicyclo[5.4.0]undec-5-en in 0,8 ml Tetrahydrofuran 15 Minuten lang gegeben. Das Gemisch wird dann während 30 Minuten bei 0° gerührt. Zugabe von 25 ml EtOAc, Waschen mit 20 ml 0.1 N wässriger HCl-Lösung und 25 ml gesättigter wässriger $NaHCO_3$-Lösung, Trocknen der organischen Phase über Natriumsulfat und Eindampfen des Lösungsmittels im Vakuum liefert die diastereomeren Trimethylsilyläther. Diese werden in 20 ml Methanol aufgelöst und mit 1 ml 2 N wässriger HCl-Lösung versetzt und 60 Minuten bei Raumtemperatur gerührt. Nach Zugabe von 168 mg (2 mMol) festem $NaHCO_3$ wird im Vakuum Rotationsverdampfer eingeengt und der Rückstand im Hochvakuum getrocknet. Chromatographie auf 12 g Merck-Kieselgel mit Toluol-EtOAc 1 : 2 (20 Fraktionen, je 8 ml) liefert ein Gemisch der beiden Titelvervindungen.

2. Das Ausgangsmaterial 1-Trimethylsilyloxy-(2R)-2-mercaptopropan kann folgendermassen erhalten werden.

Zu einem Gemisch von 920 mg des gemäss Beispiel 2, unter 2.3. hergestellten (2R)-2-Mercapto-1-propanols und 1,36 ml Trimethylchlorsilan, in 10 ml trockenem Benzol wird bei 0° unter Rühren 1,53 ml Triäthylamin innerhalb 15 Minuten gegeben. Das Gemisch wird noch 15 Minuten bei Raumtemperatur und 15 Minuten bei 60° gerührt, abgekühlt und vom entstandenen Niederschlag abfiltriert. Der Filterrückstand wird mit 10 ml Benzol gewaschen und die vereinigten Filtrate im Rotationsverdampfer bei 11 mm und 25° entfernt. Kugelrohrdestillation des Rückstands bei 11 mm und 100° ergibt reines flüssiges Produkt.

IR-Spektrum in $CH_2Cl_2$, $cm^{-1}$ : 2 950, 1 080, 875, 845. NMR in Benzol-d, ppm : 3.35, 2H, m ; 2.8, 1H, m ; 1.4, 1H, d, J = 7 ; 1.15, 3H, d, J = 7 Hz ; 0, 9H, s.

Beispiel 5

(4R)-4-((2R)-1-Hydroxyprop-2-yl-thio)-2-azetidinon und (4S)-4-((2R)-1-Hydroxyprop-2-yl-thio)-2-azetidinon.

Das diastereomere Gemisch kann durch Chromatographie aufgetrennt werden : 3,2 g wird auf 400 g Merck Kieselgel mit EtOAc-Toluol 2 : 1 (20 Fraktionen, je 300 ml) und EtOAc (10 Fraktionen, je 300 ml) teilweise aufgetrennt. Fraktionen 10-11 enthalten 410 mg reines 4(S)-Isomeres. $R_f$-Wert 0.18 (EtOAc). NMR-Spektrum in $CDCl_3$ in ppm : 7.2, 1H, breites s ; 4.90, 1H, dd, J = 3 Hz, J = 5 Hz ; 2.6-4.0, 5H, m ; 1.26, 3H, d, J = 7 Hz, Fraktionen 12-17 enthalten beide Diastereomere und Fraktionen 18-30 enthalten 400 mg reine 4(R)-Verbindung. $R_f$-Wert : 0.15 (EtOAc). NMR-Spektrum in $CDCl_3$ in ppm : 7.0, 1H, breites s ; 5.0, 1H, dd, J = 3 Hz, J = 5 Hz ; 2.5-3.8, 5H, m ; 1.35, 3H, d, J = 7 Hz.

Beispiel 6

2,2-Dimethyl-9-oxo-3-oxa-6-thia-1-azabicyclo[5.2.0$^{1,7}$]-nonan.

15 g des gemäss Beispiel 1 erhältlichen 4-(2-Hydroxyäthyltio)-2-oxo-azetidins werden in 100 ml Aethanol-freiem Methylenchlorid gelöst und bei − 10° mit 26,2 g (30,5 ml, 0.25 Mol) Acetondimethylketal versetzt. Bei − 10° wird unter Rühren 2 ml Bortrifluorid-Aetherat zugegeben und das Gemisch 30 Minuten bei − 10° 2 Stunden lang bei Raumtemperatur gerührt. Das Gemisch wird dann mit 150 ml Methylenchlorid verdünnt und mit 350 ml eiskalter wässriger gesättigter Natriumbicarbonatlösung gewaschen. Die organische Phase wird über Natriumsulfat getrocknet, filtriert und im Vakuum-Rotationsverdampfer vom Lösungsmittel befreit und dann im Hochvakuum getrocknet. Der feste Rückstand wird aus Aether-n-Hexan umkristallisiert. Smp. der racemischen Titelverbindung 67-68°. $R_f$ : 0.5 (Aethylacetat). IR-Spektrum ($CH_2Cl_2$) : Absorptionsbanden bei 1 750, 1 345, 1 240, 1 080 $cm^{-1}$.

Beispiel 7

(7R,5R)-2,2,5-Trimethyl-9-oxo-3-oxa-6-thia-1-azabicyclo[5.2.0$^{1,7}$]nonan.

166 mg des gemäss Beispiel 5 erhältlichen (4R)-4-((2R)-1-Hydroxyprop-2-yl-thio)-2-azetidinons werden in einem Gemisch von 1 ml trockenem, Aethanol-freiem Methylenchlorid und 0,31 ml Acetondimethylacetal aufgelöst. Unter Rühren bei − 10° wird 20 µl Bortrifluoridäthylätherat zugegeben und das Gemisch 30 Minuten bei − 10° und 150 Minuten bei Raumtemperatur gerührt. Verdünnen mit 10 ml Methylenchlorid, Waschen mit 10 ml gesättiger, wässriger $NaHCO_3$-Lösung, Trocknen der organischen Phase über Natriumsulfat, Filtration und Eindampfen des Lösungsmittels ergibt 225 mg eines nichtkristallinen Rückstands. Chromatographie auf 7 g Merck Kieselgel mit Toluol-EtOAc 4 : 1 (20 Fraktionen, je 3,5 ml) ergibt die reine Titelverbindung. Smp. 87° (n-Hexan). $R_f$ : 0.57 (EtOAc). NMR-Spektrum in $CDCl_3$ in ppm : 5.17, 1H, dd, J = 3, J = 5 ; 2.4-4.6, 5H, m ; 1.77, 3H, s ; 1.50, 3H, s ; 1.47, 3H, d, J = 7 Hz. $[\alpha]_D^{20}$ : + 131° ($CHCl_3$, c = 1).

Beispiel 8

(7S,5R)-2,2,5-Trimethyl-9-oxo-3-oxa-6-thia-1-azabicyclo[5.2.0$^{1,7}$]nonan.

Analog Beispiel 7 wird aus 166 mg des gemäss Beispiel 5 erhältlichen (4S)-4-((2R)-1-Hydroxyprop-2-yl-thio)-2-azetidinons reine Titelverbindung erhalten. Smp. 54° (n-Hexan). $R_f$ 0.57 (EtOAc). NMR-Spektrum in $CDCl_3$ in ppm : 5.17, 1H, dd, J = 3 Hz, J = 5 Hz ; 2.4-4.6, 5H, m ; 1.75, 3H, s ; 1.47, 3H, s ; 1.15, 3H, d, J = 7 Hz. $[\alpha]_D^{20}$ : − 133° ($CHCl_3$, c = 1).

Beispiel 9

2,2-Dimethyl-trans-8-(1-hydroxyäthyl)-9-oxo-3-oxa-6-thia-1-azabicyclo[5.2.0.$^{1,7}$]nonan.

Zu einer Lösung von 11,0 g (15,5 ml, 0.11 Mol) Diisopropylamin in 200 ml trockenem Tetrahydrofuran werden bei − 65 °C unter Rühren unter Stickstoffatmosphäre innerhalb 15 Minuten tropfenweise 55 ml einer 2.0 M Lösung von n-Butyllithium in n-Hexan (0.11 Mol) zugegeben und das Gemisch bei − 65° während 15 Minuten gerührt. Dann wird bei − 65° unter Rühren eine Lösung von 18,7 g (0.1 Mol) des gemäss Beispiel 6 erhältlichen 2,2-Dimethyl-9-oxo-3-oxa-6-thia-1-azabicyclo[5.2.0.$^{1,7}$]nonans in 80 ml trockenem Tetrahydrofuran tropfenweise innerhalb 15 Minuten zugegeben und 10 Minuten lang bei − 65° weitergerührt. Danach werden bei − 65° 15 Minuten lang 17 ml (0.3 Mol) Acetaldehyd in 80 ml trockenem Tretrahydrofuran tropfenweise zugegeben. Das Gemisch lässt man langsam auf 0° aufwärmen und 1,5 Stunden bei 0° rühren. Das Reaktionsgemisch wird auf 1 kg Eis gegossen und mit 2,5 l Methylenchlorid extrahiert, die organische Phase über Natriumsulfat getrocknet, filtriert, und das

Lösungsmittel im Vakuum-Rotationsverdampfer entfernt. Der Rückstand wird auf 1 kg Merck Kieselgel chromatographiert mit Toluol-Aethylacetat (2 : 1) als Laufmittel (zwanzig 500 ml-Fraktionen). Nach Eindampfen des Lösungsmittels ergibt sich ein viskoses Gemisch der erythro-trans-Titelverbindung (2 Teile) und der threo-trans-Titelverbindung (1 Teil). $R_f$ = 0.4 (Aethylacetat). IR-Spektrum ($CH_2Cl_2$) : Absorptionsbanden bei 3 550, 1 740, 1 220, 1 080 $cm^{-1}$.

Beispiel 10

2,2-Dimethyl-trans-8-allyl-9-oxo-3-oxa-6-thia-1-aza-bicyclo[5.2.0.$^{1,7}$]nonan.

Zu einer Lösung von 0,35 g (0,5 ml, 3,5 mMol) Disopropylamin in 5 ml trockenem Tetrahydrofuran wird bei – 70° 1,8 ml einer 2 N Lösung von Butyllithium in n-Hexan gegeben. Nach 10 Minuten wird eine Lösung von 2,2-Dimethyl-9-oxo-3-oxa-6-thia-1-azabicyclo[5.2.0.$^{1,7}$]nonan (0,56 g, 3 mMol) in 5 ml Tetrahydrofuran zugegeben. Nach weiteren 10 Minuten wird 1,1 g (0,8 ml, 9 mMol) Allylbromid zugegeben und das Gemisch 2 Stunden bei 0° gerührt. Das Gemisch wird mit 100 ml EtOAc verdünnt, mit 20 ml 1 N $NaH_2PO_4$-Lösung gewaschen, mit 20 ml Wasser nachgewaschen und die organische Phase über Natriumsulfat getrocknet. Nach Abdampfen des Lösungsmittels in Vakuum wird der Rückstand auf 25 g Merck Kieselgel chromatographiert (Toluol-EtOAc : 9 : 1) und ergibt die reine Titelverbindung. $R_f$-Wert : 0.5 (Toluol-EtOAc 1 : 1) NMR-Spektrum ($CDCl_3$ in ppm) : 2,3-6.3, 8H, m ; 1.7, 3H, S ; 1.45, 3H, S.

Beispiel 11

2,2-Dimethyl-trans-8-(1-p-nitrobenzoxycarbonyloxyäthyl)-9-oxo-3-oxa-6-thia-1-azabicyclo[5.2.0.$^{1,7}$]nonan.

Eine Lösung von 17,4 g (75 mMol) 2,2-Dimethyl-trans-8-(1-hydroxyäthyl-9-oxo-3-oxa-6-thia-1-azabicyclo[5.2.0.$^{1,7}$]nonan in 200 ml äthanolfreiem Methylenchlorid wird bei – 10° mit 21.6 g (0.1 Mol) festem Chlorameisensäure-p-nitrobenzylester versetzt und zu der Lösung innerhalb 30 Minuten bei – 10° 12,2 g (0.1 Mol) festes 4-N'N'-Dimethylaminopyridin in kleinen Portionen eingetragen. Das Reaktionsgemisch wird noch 1 Stunde bei Raumtemperatur gerührt und dann 6 Stunden am Rückfluss erwärmt. Nach Abkühlen wird mit 1,5 l Methylenchlorid verdünnt und mit 1 l kalter wässriger NaCl-Lösung gewaschen. Die organische Phase wird über Natriumsulfat getrocknet, filtriert und das Lösungsmittel im Vakuum-Rotationsverdampfer getrocknet, filtriert und das Lösungsmittel im Vakuum-Rotationsverdampfer abgezogen. Der Rückstand wird auf 1 kg Merck $SiO_2$ mit Toluol-Essigester (9 : 1) als Laufmittel (20 500 ml Fraktionen) chromatographiert. Nach Entfernen des Lösungsmittels ergibt sich ein racemisches Gemisch aus den Erythro-trans und Threo-trans-Titelverbindungen. $R_f$ = 0.4 (Toluol-Aethylacetat 1 : 1), IR-Specktrum ($CH_2Cl_2$) : Absorptionsbanden bei 1 760, 1 750, 1 610, 1 530, 1 355 $cm^{-1}$.

Beispiel 12

2,2-Dimethyl-trans-8-(1-p-nitrobenzyloxycarbonyloxyäthyl)-9-oxo-3-oxa-6-thia-1-azabicyclo[5.2.0.$^{1,7}$]nonan-6-dioxid.

Zu einer Lösung von 16,4 g des gemäss Beispiel 11 erhältlichen 2,2-Dimethyl-trans-8-(1-p-nitrobenzyloxycarbonyloxyäthyl)-9-oxo-3-oxa-6-thia-1-aza-bicyclo[5.2.0.$^{1,7}$]nonan in 200 ml trocknenem Methylenchlorid wird bei – 10° innerhalb 30 Minuten 19,4 g (0.1 Mol) m-Chlorperbenzoesäure (90 %) in kleinen Portionen gegeben.

Das Gemisch wird dann 1 Stunde bei 0° gerührt, mit 1,5 l Methylenchlorid verdünnt, mit gesättigter wässriger Natriumbicarbonatlösung, 10 %iger Natriumbisulfitlösung und nochmals mit gesättigter Natriumbicarbonatlösung gewaschen. Die organische Phase wird über Natriumsulfat getrocknet und das Lösungsmittel im Vakuum-Rotationsverdampfer eingedampft. Der nichtkristalline Rückstand besteht aus einem racemischen Gemisch der Erythro-trans und Threo-trans Titelverbindungen und wird direkt weiterverwendet. $R_f$ = 0.6 (EtOAc). IR Sepktrum ($CH_2Cl_2$) : Absorptionsbanden bei 1 780, 1 750, 1 610, 1 325, 1 135 $cm^{-1}$.

Beispiel 13

trans-3-(1-p-Nitrobenzyloxycarbonyloxyäthyl)-4-(2-hydroxyäthylsulfonyl)-2-oxo-azetidin.

17,7 g 40 mMol rohes 2,2-Dimethyl-trans-8-(1-p-nitrobenzyloxycarbonyloxyäthyl)-9-oxo-3-oxa-6-thia-1-azabicyclo[5.2.0.$^{1,7}$]nonan-6-dioxyds wird in 260 ml Eisessig aufgelöst und mit 60 ml Wasser verdünnt. Das Gemisch wird während 105 Minuten am Rückfluss erhitzt im Vakuum-Rotationsverdampfer vom Lösungsmittel befreit und im Hochvakuum getrocknet. Der Rückstand wird in 60 ml Methylenchlorid gelöst und bei – 20° kristallisiert. Durch eine weitere Umkristallisation der Kristalle wird reine erythro-trans Titelverbindung erhalten. $R_f$-Wert : 0.35 (EtOAc), Smp. 152°.

IR-Spektrum ($CH_2Cl_2$) : Absorptionsbanden bei 3 570, 3 370, 1 790, 1 750, 1 520, 1 350 $cm^{-1}$. NMR-

Spektrum in (DMSO-d/100 Mc ; in ppm) : 9.1, 1H, s ; 8.3-7.6, 4H, m ; 5.3, 2H, s ; 5.5-4.8, 1H ; 5.1, 1H, dd, J = 4 Hz, J = 6.5 Hz ; 4.9, 1H, d, J = 2.5 Hz ; 3.7-3,9, 3H, m ; 3.3-3.4, 2H, m ; 1.5, d, 3H, J = 6.5 Hz.

Die Mutterlauge wird eingedampft und der Rückstand auf 600 g Merck $SiO_2$ mit Toluol-Aethylacetat als Laufmittel (2 : 1) chromatographiert. Die Fraktionen mit $R_f$ einem Wert von 0.4 (EtOAc) werden vereinigt und das Lösungsmittel im Vakuum-Rotationsverdampfer entfernt. Die reine amorphe threo-trans-Titelverbindung hat einen $R_f$-Wert = 0.4 (EtOAc). IR-Spektrum ($CH_2Cl_2$) : Absorptionsbanden bei 3 570, 3 370, 1 790, 1 750, 1 520, 1 350 $cm^{-1}$. NMR-Spektrum in (DMSO-d/100 Mc ; in ppm) : 9.1, 1H, s ; 8.4-7.6, 4H, m ; 5.3, 2H, s ; 5.5-5.0, 1H, 5.1, 1H, dd, J = 5, J = 6.5 Hz ; 4.0, 1H, d, J = 2.5 Hz ; 3.9-3.7, 3H, m ; 3.3, 2H, m ; 1.45, 3H, d, J = 6.5 Hz.

Folgende Beispiele illustrieren die Weiterverarbeitung der gemäss Beispiel 1-13 erhältlichen Verbindungen :

### Beispiel 14

threo-trans-3-(1-p-Nitrobenzyloxycarbonyloxyäthyl)-4-(cis-β-carbomethoxyvinylmercapto)-2-oxo-azetidin.

In einer auf − 15° gekühlten Lösung von 4,73 g (24 mMol) cis-β-Carbomethoxyvinylisothiuroniumchlorid in 100 ml 95 %igem Aethanol werden unter Stickstoffatmosphäre unter Rühren 48 ml 1.0 N wässrige Natronlaugelösung innerhalb 5 Minuten zugetropft, wobei die Innentemperatur auf − 10° durch externe Kühlung gehalten wird. Nach weiterem 5-minütigem Rühren bei − 10° wird eine Lösung von 8,1 g (20 mMol) threo-trans-3-(1-p-Nitrobenzyloxycarbonyloxyäthyl)-4-(2-hydroxyäthylsulfonyl)-2-oxo-azetidin in 60 ml 95 %igem Aethanol innerhalb 5 Minuten bei − 10° zugetropft, und das Gemisch anschliessend während 80 Minuten bei 2° gerührt. Nach Zugabe von 1 l Methylenchlorid wird zweimal mit je 500 ml gesättigter wässriger NaCl-Lösung gewaschen, die organische Phase über Natriumsulfat getrocknet, filtriert und im Vakuum-Rotationsverdampfer vom Lösungsmittel befreit. Der feste Rückstand wird auf 300 g Merck-$SiO_2$ mit Toluol-Aethylacetat als Laufmittel (2 : 1) chromatographiert. Nach Abdampfen des Lösungsmittels im Vakuum wird aus Methylenchlorid n-Hexan kristallisiert und die reine Titelverbindung erhalten. $R_f$ = 0.6 (EtOAc). Smp. 164.5-165.5°. IR-Spektrum ($CH_2Cl_2$) : Absorptionsbanden bei 3 500, 1 790, 1 760, 1 710, 1 530, 1 360 $cm^{-1}$. NMR-Spektrum (in DMSO-d/100 Mc ; in ppm) : 8.8, 1H, s ; 8.3-7.6, 4H, m ; 7.5, 1H, d, J = 10 Hz ; 6.0, 1H, d, J = 10 Hz ; 5.3, 2H, s ; 5.1, 1H, d, J = 2.5 Hz ; 5.2-5.0, 1H, m ; 3.6, 3H, s ; 3.5, 1H, dd, J = 6 Hz, J = 2.5 Hz ; 1.35, 3H, d, J = 6.5 Hz.

### Beispiel 15

erythro-trans-3-(1-p-Nitrobenzyloxycarbonyloxyäthyl)-4-(cis-β-carbomethoxyvinylmercapto)-2-oxo-azetidin.

Nach der Vorschrift von Beispiel 14 wird die Titelverbindung aus dem entsprechenden erythro-trans-Ausgangsmaterial erhalten. $R_f$-Wert = 0.6 (EtOAc). Smp. 135-137.5°. IR-Spektrum ($CH_2Cl_2$) : Absorptionsbanden bei 3 400, 1 790, 1 760, 1 710, 1 530, 1 360 $cm^{-1}$. NMR-Spektrum (in DMSO-d/100 Mc ; in ppm) : 8.8, 1H, s ; 8.3-7.6, 4H, m ; 7.5, 1H, d, J = 10 Hz ; 6.0, 1H, d, J = 10 Hz ; 5.3, 2H, s ; 5.05, 1H, d, J = 2.5 Hz ; 5.2-5.0, 1H, m ; 3.65, 3H, s ; 3.55, 1H, dd, J = 2.5 Hz, J = 5 Hz ; 1.4, 3H, d, J = 6.5 Hz.

### Beispiel 16

2-[threo-trans-3-(1-p-Nitrobenzyloxycarbonyloxyäthyl)-4-cis-β-carbomethoxy vinylmercapto)-2-oxo-azetidinyl]-2-hydroxyessigsäureacetonylester.

Ein Gemisch von 2,25 g (50 mMol) threo-trans-3-(1-p-Nitro-benzyloxycarbonyloxyäthyl)-4-(cis-β-carbomethoxyvinylmercapto)-2-oxoazetidin und 2 g Glyoxylsäureacetonylester wird bei 30° in 5 ml N,N-Dimethylformamid und 10 ml Toluol gelöst und über 20 g Molekularsieb 3 Å während 15 Stunden gerührt. Nach Abkühlen des Gemisches wird filtriert, der Rückstand mit Essigester mehrmals gewaschen und die vereinigten Filtrate im Vakuum (bei 0,02 mm Hg) bei 55° Badtemperatur mehrmals mit Dimethylformamid eingedampft und dann auf 120 g Merck $SiO_2$ mit Toluol-Essigester (2 : 1) als Laufmittel chromatographiert. Die Fraktionen, welche Produkt enthalten, werden vereinigt und im Vakuum-Rotationsverdampfer vom Lösungsmittel befreit. Die amorphe, viskose Masse hat $R_f$ = 0.6 (EtOAc) ; IR-Spektrum ($CH_2Cl_2$) : Absorptionsbanden bei 3 500, 1 780, 1 760, 1 740, 1 530 $cm^{-1}$.

### Beispiel 17

2-[erythro-trans-3-(1-p-Nitrobenzyloxycarbonyloxyäthyl)-4-cis-β-carbomethoxyvinylmercapto)-2-oxo-azetidinyl]-2-hydroxyessigsäureacetonylester.

Nach der Vorschrift von Beispiel 16 wird aus dem erythro-trans-Ausgangsmaterial die entsprechende Titelverbindung erhalten. Amorphe, viskose Masse $R_f$ = 0.6 (EtOAc) ; IR-Spektrum ($CH_2Cl_2$) : Absorptionsbanden bei 3 500, 1 780, 1 760, 1 740, 1 530 $cm^{-1}$.

Beispiel 18

2-[threo-trans-3-(1-p-Nitrcbenzyloxycarbonyloxyäthyl)-4-(cis-β-carbomethoxyvinylmercapto)-2-oxo-azetidinyl]-2-triphenylphosphoranylidenessigsäureacetonylester.

Zu einer Lösung von 2,7 g (5 mMol) 2-[threo-trans-3-(1-p-Nitrobenzyloxycarbonyloxyäthyl)-4-(cis-β-carbomethoxyvinylmercapto)-2-oxo-azetidinyl]-2-hydroxyessigsäureacetonylester in 25 ml trockenem Tetrahydrofuran werden unter Stickstoffatmosphäre und unter Rühren bei – 10° 0,43 ml (6 mMol) Thionylchlorid und dann bei – 10° 2 Minuten lang tropfenweise 0,83 ml (6 mMol) Triäthylamin zugegeben.

Das Gemisch wird noch 30 Minuten lang bei 0° gerührt, mit 150 ml eiskaltem Methylenchlorid versetzt und anschliessend mit 50 ml 0.1 N wässriger HCl-Lösung, dann mit 50 ml gesättigter Kochsalzlösung gewaschen. Die organische Phase wird über Natriumsulfat getrocknet, filtriert und das Lösungsmittel im Vakuum-Rotationsverdampfer entfernt. Der amorphe feste Rückstand wird direkt weiterverwendet.

Der amorphe feste Rückstand (2,8 g, 5 mMol) wird in einem Gemisch von 2,9 g (12 mMol) Triphenylphosphin und 4 ml Tetrahydrofuran gelöst und unter Stickstoffatmosphäre 24 Stunden bei Raumtemperatur stehen gelassen. Das Gemisch wird mit 100 ml Methylenchlorid verdünnt, und zweimal mit je 30 ml Portionen 10 %iger wässriger Sodalösung gewaschen. Die organische Phase wird über Natriumsulfat getrocknet, filtriert und im Vakuum-Rotationsverdampfer vom Lösungsmittel befreit. Der Rückstand wird auf 100 g Merck $SiO_2$ mit Toluol-Essiggester (3 : 1) als Laufmittel chromatographiert. Der amorphe feste Rückstand hat einen $R_f$-Wert = 0.5 (EtOAc). IR-Spektrum ($CH_2Cl_2$) : Absorptionsbanden 1 755, 1 695, 1 630, 1 525 $cm^{-1}$.

Beispiel 19

2-[erythro-trans-3-(1-p-Nitrobenzyloxycarbonyloxyäthyl)-4-(cis-β-carbomethoxyvinylmercapto)-2-oxo-azetidinyl]-2-triphenylphosphoranylidenessigsäureacetonylester.

Nach der Vorschrift von Beispiel 18 wird aus dem erythro-trans-Ausgangsmaterial auch die entsprechende Titelverdingung erhalten. Amorpher Festkörper, $R_f$ = 0.5 (EtOAc), IR-Spektrum ($CH_2Cl_2$) : Absorptionsbanden bei 1 755, 1 695, 1 630, 1 525 $cm^{-1}$.

Beispiel 20

threo-trans-6-(1-p-Nitrobenzyloxycarbonyloxyäthyl)-penem-3-carbonsäureacetonylester.

Eine Lösung von 1,96 g (2.5 mMol) 2-[threo-trans-3-(1-p-Nitrobenzyloxycarbonyloxyäthyl)-4-(cis-β-carbomethoxyvinylmercapto)-2-oxo-azetidinyl]-2-triphenylphosphoranylidenessigsäureacetonylester in 40 ml Methylenchlorid wird bei – 20° mit 1,25 ml Trifluoressigsäure versetzt und in das Gemisch bei dieser Temperatur während 15 Minuten ein Strom von $O_3$ in $O_2$ (0.33 mMol $O_3$ pro Minute) geleitet. Anschliessend wird überschüssiges $O_3$ durch Einleiten von Stickstoff entfernt, 2 ml Dimethylsulfid zugegeben, bei 10° 10 Minuten lang gerührt, mit 50 ml eiskaltem Methylenchlorid verdünnt, zweimal mit 25 ml Portionen von 10 %iger wässriger Kaliumbicarbonatlösung gewaschen, die organische Phase über Natriumsulfat getrocknet, im Vakuum-Rotationsverdampfer eingedampft, der feste Rückstand im Hochvakuum-Rotationsverdampfer eingedampft, der feste Rückstand im Hochvakuum 2 Minuten getrocknet und in 60 ml Methylenchlorid (frisch über Alox filtriert) gelöst. Die Lösung wird dann während 90 Minuten am Rückfluss unter Stickstoffatmosphäre erhitzt, nach Abkühlen im Vakuum-Rotationsverdampfer eingedampft und der Rückstand auf 25 g Merck $SiO_2$ mit Toluol-Aethylacetat (3 : 1) als Laufmittel chromatographiert. Nach dem Eindampfen der geeigneten Fraktionen ergibt sich die reine amorphe Titelverbindung. $R_f$-Wert = 0.55 (EtOAc). IR-Spektrum ($CH_2Cl_2$) : Absorptionsbanden bei 1 795, 1 750, 1 745, 1 720, 1 530, 1 350 $cm^{-1}$. NMR-Spektrum (in Aceton-d/100 Mc ; in ppm) : 8.3-7.6, 4H, m ; 7.6, 1H, s ; 5.95, 1H, d, J = 2.5 Hz ; 5.3, 2H, s ; 5.4-5.1, 1H, m ; 4.8, 2H, s ; 4.2, 1H, dd, J = 6 Hz, J = 2.5 Hz ; 2.1, 3H, s ; 1.45, 3H, d, J = 6.5 Hz.

Beispiel 21

erythro-trans-6-(1-p-Nitrobenzyloxycarbonyloxyäthyl)-penem-3-carbonsäureacetonylester.

Nach der Vorschrift von Beispiel 20 wird ausgehend von der erythro-trans-Ausgangsverbindung auch die entsprechende Titelverbindung erhalten. Smp. 154-156°, $R_f$ = 0.55 (EtOAc). IR-Spektrum ($CH_2Cl_2$) : Absorptionsbanden bei 1 795, 1 745, 1 720, 1 530, 1 350 $cm^{-1}$. NMR-Spektrum (in Aceton-d/100 Mc ; ppm) : 8.4-7.6, 4H, m ; 7.6, 1H, s ; 5.8, 1H, d, J = 2.5 Hz ; 5.35, 2H, s ; 5.25, 1H, dd, J = 6.5, J = 3.5 Hz ; 4.80, 2H, s ; 3.8, 1H, m ; 2.1, 3H, s ; 1.5, 3H, d, J = 6.5 Hz.

Beispiel 22

threo-trans-6-(1-Hydroxyäthyl)-penem-3-carbonsäureacetonylester).

Eine Lösung von 0,936 g (2 mMol) threo-trans-6-(1-p-Nitrobenzyloxycarbonyloxyäthyl)-penem-3-carbonsäureacetonylester in 80 ml eines Gemisches aus gleichen Teilen Acetonitril und 95 %igem Aethanol wird über 800 mg 10 %igem Pd/C-Katalysator bei Raumtemperatur während 2,5 Stunden hydriert. Das Gemisch wird filtriert und das Filtrat eingedampft und der Rückstand auf 40 g Merck $SiO_2$ mit Toluöl-Aethylacetat 2 : 1 als Laufmittel chromatographiert. Die geeigneten Fraktionen werden vom Lösungsmittel im Vakuum-Rotationsverdampfer befreit und der Rückstand durch Zugabe von Methylenchlorid-Aether kristallisiert. $R_f$-Wert = 0.4 (EtOAc), Smp. 115-116°. IR-Spektrum ($CH_2Cl_2$) : 3 580, 1 790, 1 725, 1 720, 1 560, 1 175 $cm^{-1}$. NMR-Spektrum (in Aceton-d/100 Mc/ppm) : 7.6, 1H, s ; 5.85, 1H, d, J = 2 Hz ; 4.8, 1H, s ; 4.4, 1H, m ; 4.4-4.0, 1H, s ; 3.85, 1H, dd, J = 7 Hz, J = 2 Hz ; 2.15, 3H, s ; 1.3, 3H, d, J = 6.5 Hz.

Beispiel 23

erythro-trans-6-(1-Hydroxyäthyl)-penem-3-carbonsäureacetonylester.

Nach der Vorschrift von Beispiel 22 wird aus der erythro-trans-Ausgangsverbindung auch die entsprechende Titelverbindung erhalten. $R_f$ = 0.4 (EtOAc), Smp. 120-121.5°. IR-Spektrum ($CH_2Cl_2$) : Absorptionsbanden bei 3 580, 1 790, 1 725, 1 720, 1 560, 1 175 $cm^{-1}$. NMR-Spektrum (in Aceton-d/100 Mc/ppm) : 7.55, 1H, d, J = 1 Hz ; 5,8, 1H, d, J = 2 Hz ; 4.8, 2H, s ; 4.4-4.0, 1H, s ; 4.4-4.2, 1H, m ; 4.0, 1H, m ; 2.15, 3H, s ; 1.35, 3H, d, J = 6.5 Hz.

Beispiel 24

threo-trans-6-(1-Hydroxyäthyl)-penem-3-carbonsäure.

Zu einer Lösung von 0,271 g (1 mMol) threo-trans-6-(1-Hydroxyäthyl)-penem-3-carbonsäureacetonylester in 40 ml Acetonitril und 10 ml Wasser wird bei 0° unter Stickstoffatmosphäre während 15 Minuten 10 ml wässrige 0.1 N NaOH-Lösung getropft. Das Gemisch wird noch 30 Minuten bei 0° weitergerührt, dann mit 6 g aufgequollenem schwach saurem Kationenaustauscher IV (Merck) während 5 Minuten bei 0° gerührt, filtriert und im Vakuum auf ein Volumen von ca. 20 ml eingedampft und auf zehn 20 cm 20 cm Antecgel-Dodecyltrichlorsilan-DC-Platten mit Wasser chromatographiert. Eluieren mit Acetonitril $H_2O$ (3 : 1) und Lyophilisation des Filtrats ergibt das Natriumsalz der Titelverbindung als amorphen Festkörper. $R_f$-Wert = 0.4 ($H_2O$, Antecgel-Dodecyltrichlorsilan-DC-Platte). IR-Spektrum (KBr) ; 1 750 $cm^{-1}$ ; NMR-Spektrum (in $D_2O$/100 Mc/ppm) : 7.25, 1H, s ; 5.95, 1H, d, J = 2 Hz ; 4.45, 1H, m ; 4.15 1H, dd, J = 6 Hz, J = 2 Hz ; 1.48, 3H, d, J = 6.5 Hz. 12 mg des reinen Natriumsalzes werden in 0,5 ml Wasser aufgelöst und die erhaltene Lösung auf eine Ionenaustauscher-Säule (stark saurer Ionenaustauscher I, Merck), die 0.5 g gewaschenes Harz enthält, aufgetragen. Elution mit Wasser bis zur Neutralität und Lyophilisation der sauren Fraktionen liefert die reine Titelverbindung. Sie wird aus heissem Acetonitril umkristallisiert. Smp. > 230° (Umwandlung bei 155-156°). $R_f$-Wert : 0.1 (Eisessig, Toluol, Wasser 5 : 5 : 1). IR-Spektrum (KBr) : 3 480, 1 795, 1 670, 1 545, 1 435, 1 255, 1 160 $cm^{-1}$. UV-Spektrum in Aethanol : $\lambda_{max}$ 260 nm (ε 3 850), 311 nm (ε 6 400).

Beispiel 25

erythro-trans-6-(1-Hydroxyäthyl)-penem-3-carbonsäure.

Nach der Vorschrift von Beispiel 24 wird aus der erythro-trans-Ausgangsverbindung das entsprechende Natriumsalz der Titelverbindung erhalten. Amorpher Festkörper, $R_f$ = 0.4 ($H_2O$, Antecgel-Dodecyltrichlorsilan-DC-Platten). IR-Spektrum (KBr) ; 1 750 $cm^{-1}$ ; NMR-Spektrum (in $D_2O$/100 Mc/ppm) : 7.3, 1H, s ; 5.9, 1H, d, J = 2 Hz ; 4.4, 1H, m ; 4.2, 1H, dd, J = 4 Hz, J = 2 Hz ; 1.52, d, J = 6.5 Hz. Ebenso wird die reine Titelverbindung erhalten. Smp. 230° (Umwandlung bei 147-151°). $R_f$-Wert 0.1 (Eisessig, Toluol, Wasser 5 : 5 : 1). IR-Spektrum (KBr) : 3 510, 1 785, 1 670, 1 550, 1 435, 1 260, 1 180, 1 035, 835 $cm^{-1}$. UV-Spektrum in Aethanol : $\lambda_{max}$ 260 nm (ε 3 650), 311 nm (ε 6 300).

Beispiel 26

Ausgehend von ensprechenden Ausgangsmaterialien und über entsprechende Zwischenprodukte werden analog den voranstehenden Beispielen die folgenden Verbindungen erhalten :

6-Aethyl-2-(2-aminoäthylthio)-2-penem-3-carbonsäure,
6-Hydroxymethyl-2-penem-3-carbonsäure,

6-Hydroxymethyl-3-methyl-2-penem-3-carbonsäure,
6-Hydroxymethyl-2-(3-aminopropyl)-2-penem-3-carbonsäure,
6-Hydroxymethyl-2-(3-acetylaminopropyl)-2-penem-3-carbonsäure,
6-Hydroxymethyl-2-äthylthio-2-penem-3-carbonsäure,
6-Hydroxymethyl-2-(2-aminoäthylthio)-2-penem-3-carbonsäure,
6-Hydroxymethyl-2-(2-acetylaminoäthylthio-2-penem-3-carbonsäure,
6-(1-Hydroxyäthyl-2-methyl-2-penem-3-carbonsäure,
6-(1-Hydroxyäthyl-2-(3-aminopropyl-2-penem-3-carbonsäure,
6-(1-Hydroxyäthyl-2-(3-acetylaminopropyl)-2-penem-3-carbonsäure,
6-(1-Hydroxyäthyl-2-äthylthio-2-penem-3-carbonsäure,
6-(1-Hydroxyäthyl-2-(2-aminoäthylthio)-2-penem-3-carbonsäure,
6-(1-Hydroxyäthyl-2-(2-acetylaminoäthylthio)-2-penem-3-carbonsäure,
6-(2-Hydroxyprop-2-yl-2-penem-3-carbonsäure,
6-(2-Hydroxyprop-2-yl)-2-methyl-2-penem-3-carbonsäure,
6-(2-Hydroxyprop-2-yl)-2-(3-aminopropyl)-2-penem-3-carbonsäure,
6-(2-Hydroxyprop-2-yl)-2-(3-acetylaminopropyl)-2-penem-3-carbonsäure,
6-(2-Hydroxyprop-2-yl)-2-äthylthio-2-penem-3-carbonsäure,
6-(2-Hydroxyprop-2-yl)-2-(2-aminoäthylthio)-2-penem-3-carbonsäure,
6-(2-Hydroxyprop-2-yl)-2-(2-acetylaminoäthylthio)-2-penem-3-carbonsäure,
6-Hydroxymethyl-2-(1-methyl-1H-tetrazol-5-yl-thiomethyl)-2-penem-3-carbonsäure
sowohl in racemischer, als auch in optisch aktiver Form, und ihre Salze.

Beispiel 27

Trockenampullen oder Vials, enthaltend 0,5 g 6-(1-Hydroxyäthyl)-2-penem-3-carbonsäure Natriumsalz als Wirksubstanz werden wie folgt hergestellt :

Zusammensetzung

(für 1 Ampulle oder Vial) :

| | |
|---|---|
| Wirksubstanz | 0,5 g |
| Mannit | 0,05 g. |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Beispiel 28

Trockenampullen oder Vials, enthaltend 0,25 g 6-(1-Hydroxyäthyl)-2-penem-3-carbonsäure als Wirksubstanz werden wie folgt hergestellt :

Zusammensetzung

(für 1 Ampulle oder Vial) :

| | |
|---|---|
| Wirksubstanz | 0,25 g |
| Mannit | 0,025 g. |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

**Ansprüche**

1. Verfahren zur Herstellung von Verbindungen der Formel

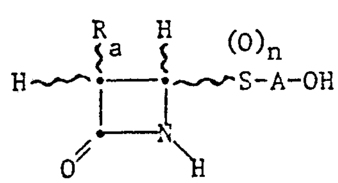

(V)

worin $R_a$ einen über ein Kohlenstoffatom mit dem Ringkohlenstoffatom verbundenen, gesättigten oder ungesättigten, gegebenenfalls substituierten, aliphatischen, cycloaliphatischen, cycloaliphatisch-aliphatischen, aromatischen oder araliphatischen Kohlenwasserstoffrest mit bis zu 18, bevorzugt bis zu 10, Kohlenstoffatomen oder einen gegebenenfalls substituierten Heterocyclyl- oder Heterocyclylniederalkylrest mit bis zu 10 Kohlenstoffatomen und 1 bis 4 Heteroatomen aus der Gruppe Stickstoff, Sauerstoff und/oder Schwefel darstellt, und worin die Substituenten von diesen Resten $R_a$ Hydroxy, Niederalkoxy, Niederalkanoyloxy, in Salzform vorliegendes Hydroxysulfonyloxy, Halogen, Mercapto, Niederalkylmercapto, Carboxyl, Niederalkoxycarbonyl, Carbamoyl, Cyan, Nitro, in Salzform vorliegendes Sulfo, oder gegebenenfalls durch Niederalkyl oder Acyl mono- oder disubstituiertes oder durch Niederalkylen disubstituiertes Amino sind, A einen Niederalkylenrest mit 2-3 Kohlenstoffatomen zwischen dem Schwefel und Sauerstoff und n 0 oder 2 bedeutet, deren Stereoisomeren und Mischungen von diesen Stereoisomeren, dadurch gekennzeichnet, dass man ein Acyloxyazetidinon der Formel

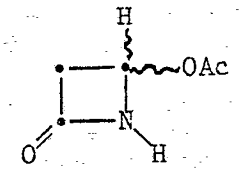

(IV)

worin Ac einen Acylrest darstellt, mit einem Hydroxyniederalkylmercaptan der Formel HS—A—OH in Gegenwart einer geeigneten Base umsetzt und gewünschtenfalls in einer erhältlichen Verbindung die Gruppe —S—A—OH in einer andere Gruppe —S—A—OH überführt und eine erhältliche Verbindung der Formel

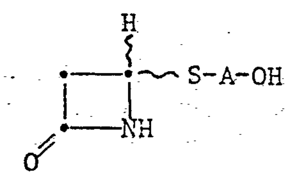

(II)

worin A die unter Formel V genannte Bedeutung hat, mit einer Carbonylverbindung der Formel

$$R_b—(C=O)—R_c \qquad (III)$$

worin jeder der Reste $R_b$ und $R_c$ Wasserstoff oder einen organischen Rest darstellt, der mit einem Kohlenstoffatom mit der Carbonylgruppe verknüpft ist, wobei $R_b$ und $R_c$ untereinander verknüpft sein können, oder einem Derivat davon, in Gegenwart eines sauren Reagenz umsetzt und in einer erhältlichen Verbindung der Formel

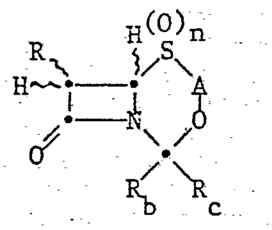

(I)

worin R Wasserstoff bedeutet, A die unter Formel V, $R_b$ und $R_c$ die unter Formel III genannten Bedeutungen haben und n 0 bedeutet, einen Rest $R_a$ einführt und gewünschtenfalls eine erhältliche Verbindung in eine Verbindung der Formel I überführt, worin n für 2 steht und/oder ein verfahrensgemäss erhältliches Isomerengemisch in die einzelnen Isomere auftrennt, und die Verbindung der Formel I in Gegenwart eines geeigneten Solvolysereagenz solvolysiert und gewünschtenfalls eine erhältliche Verbindung der Formel V, worin n für 0 steht, in eine Verbindung der Formel V überführt, worin n für 2 steht, und/oder eine erhältliche Verbindung in die einzelnen Isomere auftrennt.

2. Verfahren zur Herstellung von Verbindungen der Formel V, worin $R_a$, A und n die in Anspruch 1 genannten Bedeutungen haben, dadurch gekennzeichnet, dass man ein Acyloxyazetidinon der Formel IV, worin Ac einen Acylrest darstellt, mit einem Hydroxyniederalkylmercaptan der Formel HS—A—OH in Gegenwart eine geeigneten Base umsetzt und gewünschtenfalls in einer erhältlichen Verbindung die Gruppe —S—A—OH in eine andere Gruppe —S—A—OH überführt und eine erhältliche Verbindung der Formel II, worin A die in Anspruch 1 unter Formel V genannte Bedeutung hat, mit einer Carbonylverbindung der Formel III worin $R_b$ und $R_c$ die in Anspruch 1 unter Formel III genannten Bedeutungen haben, oder ein Derivat davon in Gegenwart eines sauren Reagenz umsetzt und in einer erhältlichen

Verbindung der Formel I worin R Wasserstoff bedeutet, A die in Anspruch 1 unter Formel V und $R_b$ und $R_c$ die in Anspruch 1 unter Formel III genannten Bedeutungen haben und n 0 bedeutet, einen Rest $R_a$ einführt und gewünschtenfalls eine erhältliche Verbindung in eine Verbindung der Formel I überführt, worin n für 2 steht, und/oder ein verfahrensgemäss erhältliches Isomerengemisch in die einzelnen Isomere auftrennt und die Verbindung der Formel I in Gegenwart eines geeigneten Solvolysereagenz solvolysiert und gewünschtenfalls eine erhältliche Verbindung der Formel V, worin n für 0 steht, in eine Verbindung der Formel V überführt, worin n für 0 steht, in eine Verbindung der Formel V überführt, worin n für 2 steht und/oder eine verfahrensgemäss erhältliche Verbindung in die einzelnen Isomere auftrennt.

3. Verfahren nach Anspruch 1 zur Herstellung von trans-3-(1-p-Nitrobenzyloxycarbonyloxyäthyl)-4-(2-hydroxyäthylsulfonyl)-2-oxo-azetidin der Formel V, dadurch gekennzeichnet, dass man 4-Acetoxyazetidin-2-on mit 2-Mercaptoäthanol in Gegenwart einer geeigneten Base umsetzt, das erhältliche 4-(2-Hydroxyäthylthio)-2-oxo-azetidin mit Acetondimethylketal in Gegenwart eines sauren Reagenz umsetzt, in dem erhältlichen 2,2-Dimethyl-9-oxo-3-oxa-6-thia-1-azabicyclo[5.2.$0^{1,7}$]nonan den 1-Hydroxyäthylrest einführt, das erhältliche 2,2-Dimethyl-trans-8-(1-hydroxyäthyl)-9-oxo-3-oxa-6-thia-1-azabicyclo[5.2.$0^{1,7}$]nonan-6-dioxid überführt und diese Verbindung in Gegenwart eines geeigneten Solvolysereagenz solvolysiert.

4. Verfahren nach Anspruch 3 zur Herstellung von trans-3-(1-p-Nitrobenzyloxycarbonyloxyäthyl)-4-(2-hydroxyäthylsulfonyl)-2-oxo-azetidin der Formel V, dadurch gekennzeichnet, dass man 4-Acetoxyazetidin-2-on mit 2-Mercaptoäthanol in Gegenwart einer wässrigen Natriumhydroxidlösung umsetzt, das erhältliche 4-(2-Hydroxyäthylthio)-2-oxo-azetidin mit Acetondimethylketal in Gegenwart von Bortrifluoridätherat umsetzt, in dem erhältlichen 2,2-Dimethyl-9-oxo-3-oxa-6-thia-1-azabicyclo[5.2.$0^{1,7}$]nonan den Hydroxyäthylrest einführt und das erhältliche 2,2-Dimethyl-trans-8-(1-hydroxyäthyl)-9-oxo-3-oxa-6-thia-1-azabicyclo[5.2.$0^{1,7}$]nonan in das 2,2-Dimethyl-trans-8-(p-nitro-benzyloxycarbonyloxyäthyl)-9-oxo-3-oxa-6-thia-1-azabicyclo[5.2.$0^{1,7}$]nonan-6-dioxid überführt und diese Verbindung in Gegenwart von Eisessig solvolysiert.

5. Verfahren zur Herstellung von Verbindungen der Formel I, worin $R_a$ und A die in Anspruch 1 unter Formel V genannten Bedeutungen und $R_b$ und $R_c$ die in Anspruch 1 unter Formel III genannten Bedeutungen haben und n 0 bedeutet, dadurch gekennzeichnet, dass man in einer Verbindung der Formel I, worin R Wasserstoff bedeutet, A die in Anspruch 1 unter Formel V genannte Bedeutung hat und $R_b$ und $R_c$ die in Anspruch 1 unter Formel III genannten Bedeutungen haben und n 0 bedeutet, einen Rest $R_a$ einführt.

6. Verbindungen der Formel V gemäss Anspruch 1, worin $R_a$, A und n die in Anspruch 1 genannten Bedeutungen haben, deren Stereoisomere und Mischungen von diesen Stereoisomeren.

7. Verbindungen der Formel V gemäss Anspruch 1, worin $R_a$, A und n die in Anspruch 1 genannten Bedeutungen haben.

8. Verbindungen gemäss Anspruch 6 der Formel V, worin $R_a$ Niederalkyl, Niederalkenyl, Hydroxyniederalkyl, Niederalkoxyniederalkyl, Niederalkanoyloxyniederalkyl, gegebenenfalls durch Niederalkyl, Niederalkoxy, Halogen und/oder Nitro substituiertes Arylniederalkoxycarbonyloxyniederalkyl, oder in Salzform vorliegendes Hydroxysulfonyloxyniederalkyl, A Aethylen oder 1,2-Propylen und n 0 oder 2 bedeuten, die Stereoisomere von Verbindungen der Formel V und Mischungen von diesen Stereoisomeren.

9. Verbindungen gemäss Anspruch 7 der Formel V, worin $R_a$ die in Anspruch 8 genannten Bedeutungen hat, A Aethylen und n 0 oder 2 bedeutet.

10. Verbindungen gemäss Anspruch 8 der Formel V, worin $R_a$ Niederalkyl mit bis zu 4 Kohlenstoffatomen, Hydroxyniederalkyl mit bis zu 4 Kohlenstoffatomen, Niederalkenyl mit bis zu 4 Kohlenstoffatomen, Niederalkoxyniederalkyl, worin Niederalkyl und Niederalkoxy je bis zu 4 Kohlenstoffatome enthalten, Niederalkanoyloxyniederalkyl, worin Niederalkanoyloxy und Niederalkyl je bis zu 4 Kohlenstoffatome enthalten, gegebenenfalls durch Halogen und/oder Nitro substituiertes Phenylniederalkoxycarbonyloxyniederalkyl, oder in Salzform vorliegendes Hydroxysulfonyloxyniederalkyl, worin Niederalkyl bis zu 4 Kohlenstoffatome enthält, A Aethylen oder 1,2-Propylen, und n 0 oder 2 bedeuten, die Stereoisomere von Verbindungen der Formel I und Mischungen von diesen Stereoisomeren.

11. Verbindungen gemäss Anspruch 9 der Formel V, worin $R_a$ die in Anspruch 10 genannten Bedeutungen hat, A Aethylen und n 0 oder 2 bedeutet.

12. Verbindungen gemäss Anspruch 10 der Formel V, worin $R_a$ Niederalkyl mit bis zu 4 Kohlenstoffatomen, Niederalkenyl mit bis zu 4 Kohlenstoffatomen, 1-Hydroxyniederalkyl mit bis zu 4 Kohlenstoffatomen oder gegebenenfalls durch Halogen und/oder Nitro substituiertes Phenylniederalkoxycarbonyloxyniederalkyl, A Aethylen oder 1,2-Propylen und n 0 oder 2 bedeuten, die Stereoisomere von Verbindungen der Formel I und Mischungen von diesen Stereoisomeren.

13. Verbindungen gemäss Anspruch 11 der Formel V, worin $R_a$ die in Anspruch 12 genannten Bedeutungen hat, A Aethylen und n 0 oder 2 bedeuten.

14. Trans-3-(1-p-Nitrobenzyloxycarbonyloxyäthyl)-4-(2-hydroxyäthylsulfonyl)-2-oxo-azetidin.

15. Verbindungen der Formel I gemäss Anspruch 1, worin R Wasserstoff oder $R_a$ bedeutet und worin $R_a$, A und n die in Anspruch 1 unter Formel V genannten Bedeutungen und $R_b$ und $R_c$ die in Anspruch I unter Formel III genannten Bedeutungen haben, deren Stereoisomere und Mischungen von diesen Stereoisomeren.

16. Verbindungen der Formel I gemäss Anspruch 1, worin R Wasserstoff oder $R_a$ bedeutet und worin $R_a$, A und n die in Anspruch 1 unter Formel V genannten Bedeutungen und $R_b$ und $R_c$ die in Anspruch 1 unter Formel III genannten Bedeutungen haben.

17. Verbindungen gemäss Anspruch 15 der Formel I, worin R Wasserstoff oder $R_a$ bedeutet und worin $R_a$, A und n die in Anspruch 8 genannten Bedeutungen haben, und jeder der Reste $R_b$ und $R_c$ Wasserstoff, Niederalkyl, Phenylniederalkyl oder Phenyl oder, wenn zusammengenommen, Niederalkylen mit 3 bis 6 Kohlenstoffatomen bedeuten, deren Stereoisomere und Mischungen von diesen Stereoisomeren.

18. Verbindungen gemäss Anspruch 16 der Formel I, worin R Wasserstoff oder $R_a$ bedeutet und worin $R_a$ die in Anspruch 8 genannten Bedeutungen hat und A Aethylen und n oder 2 bedeuten und $R_b$ und $R_c$ die in Anspruch 17 genannten Bedeutungen haben.

19. Verbindungen gemäss Anspruch 17 der Formel I, worin R Wasserstoff oder $R_a$ bedeutet und worin $R_a$, A und n die in Anspruch 10 genannten Bedeutungen haben, und jeder der Reste $R_b$ und $R_c$ Wasserstoff oder Niederalkyl mit bis zu 4 Kohlenstoffatomen oder, wenn zusammengenommen. Niederalkylen mit 3-6 Kohlenstoffatomen bedeuten, deren Stereoisomere und Mischungen von diesen Stereoisomeren.

20. Verbindungen gemäss Anspruch 18 der Formel I, worin R Wasserstoff oder $R_a$ bedeutet und worin $R_a$ die in Anspruch 10 genannten Bedeutungen hat und A Aethylen und n 0 oder 2 bedeuten und $R_b$ und $R_c$ die in Anspruch 19 genannten Bedeutungen haben.

21. Verbindungen gemäss Anspruch 19 der Formel I, worin R Wasserstoff oder $R_a$ bedeutet und worin $R_a$, A und n die in Anspruch 12 genannten Bedeutungen haben, jeder der Rest $R_b$ und $R_c$ Niederalkyl oder, wenn zusammengenommen, Niederalkylen mit 3 bis 6 Kohlenstoffatomen darstellt, deren Stereoisomere und Mischungen von diesen Stereoisomeren.

22. Verbindungen gemäss Anspruch 20 der Formel I, worin R Wasserstoff oder $R_a$ bedeutet und worin $R_a$ die in Anspruch 12 genannten Bedeutungen hat und A Aethylen und n 0 oder 2 bedeuten und $R_b$ und $R_c$ die in Anspruch 21 genannten Bedeutungen haben.

23. 2,2-Dimethyl-9-oxo-3-oxa-6-thia-1-azabicyclo[5.2.0$^{1.7}$]-nonan.

24. (7R,5R)-2,2,5-Trimethyl-9-oxo-3-oxa-6-thia-1-azabicyclo-[5.2.0$^{1.7}$]-nonan.

25. (7S,5R)-2,2,5-Trimethyl-9-oxo-3-oxa-6-thia-1-azabicyclo-[5.2.0$^{1.7}$]nonan.

26. 2,2-Dimethyl-trans-8-(1-hydroxyäthyl)-9-oxo-3-oxa-6-thia-1-azabicyclo[5.2.0$^{1.7}$]nonan.

27. 2,2-Dimethyl-trans-8-allyl-9-oxo-3-oxa-3-oxa-6-thia-1-azabicyclo-[5.2.0$^{1.7}$]nonan.

28. 2,2-Dimethyl-trans-8-(1-p-nitrobenzoxycarbonyloxyäthyl)-9-oxo-3-oxa-6-thia-1-azabicyclo[5.2.0$^{1.7}$] nonan.

29. 2,2-Dimethyl-trans-8-(1-p-nitrobenzoxycarbonyloxyäthyl)-9-oxo-3-oxa-6-thia-1-azabicyclo[5.2.0$^{1.7}$] nonan-6-dioxid.

30. Verbindungen der Formel

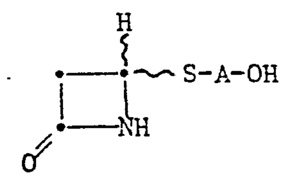

(II)

worin A einen Niederalkylenrest mit 2-3 Kohlenstoffatomen darstellt, die Stereoisomere von Verbindungen der Formel II und Mischungen von diesen Stereoisomeren.

31. Verbindungen der Formel II, worin A einen Niederalkylenrest mit 2-3 Kohlenstoffatomen darstellt.

32. 4-(2-Hydroxyäthylthio)-2-oxo-azetidin.

33. (4R)-4-((2R)-1-Hydroxyprop-2-yl-thio)-2-azetidinon.

34. (4S)-4-((2R)-1-Hydroxyprop-2-yl-thio)-2-azetidinon.

35. Die nach dem Verfahren gemäss Anspruch 1 erhältlichen Verbindungen.

36. Die nach dem Verfahren gemäss Anspruch 5 erhältlichen Verbindungen.

## Claims

1. Process for the production of compounds of the formula

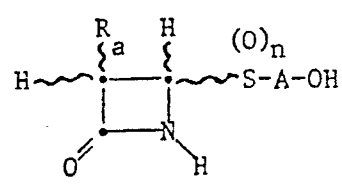

(V)

wherein $R_a$ represents a saturated or unsaturated, unsubstituted or substituted, aliphatic, cycloaliphatic,

cycloaliphatic-aliphatic, aromatic or araliphatic hydrocarbon radical bonded to the ring carbon atom by way of a carbon atom, and having up to 18, preferably up to 10, carbon atoms, or an unsubstituted or substituted heterocyclyl or heterocyclyl-lower-alkyl radical having up to 10 carbon atoms and 1 to 4 hetero atoms from the group comprising nitrogen, oxygen and/or sulfur, and wherein the substituents of these radicals $R_a$ are hydroxyl, lower alkoxy, lower alkanoyloxy, hydroxysulfonyloxy in salt form, halogen, mercapto, lower alkylmercapto, carboxyl, lower alkoxycarbonyl, carbamoyl, cyano, nitro, sulfo in salt form, or amino unsubstituted or mono- or disubstituted by lower alkyl or acyl, or disubstituted by lower alkylene, A represents a lower alkylene radical having 2-3 carbon atoms between the sulfur and oxygen atoms, and n is 0 or 2, of stereoisomers thereof and of mixtures of these stereoisomers, characterised in that an acyloxyazetidinone of the formula

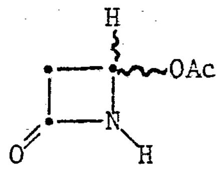

(IV)

wherein Ac represents an acyl radical, is reacted with a hydroxy-lower-alkyl mercaptan of the formula HS—A—OH in the presence of a suitable base and, if desired, in a resulting compound the —S—A—OH group is converted into a different —S—A—OH group, and a resulting compound of the formula

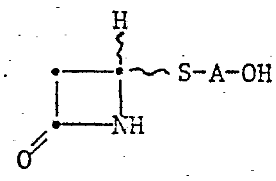

(II)

wherein A has the meaning defined under the formula V, is reacted with a carbonyl compound of the formula

$$R_b—(C=O)—R_c \quad \text{(III)}$$

wherein each of the radicals $R_b$ and $R_c$ represents hydrogen or an organic radical bonded by way of a carbon atom to the carbonyl group, it being possible for $R_b$ and $R_c$ to be bonded to one another, or with a derivative thereof, in the presence of an acid reagent, and in a resulting compound of the formula

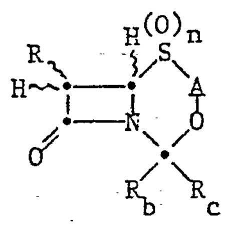

(I)

wherein R is hydrogen, A has the meaning given under the formula V, $R_b$ and $R_c$ have the meanings given under the formula III, and n is zero, a radical $R_a$ is introduced and, if desired, a resulting compound is converted into a compound of the formula I wherein n is 2, and/or an isomeric mixture obtainable according to the process is separated into the individual isomers, and the compound of the formula I is solvolysed in the presence of a suitable solvolysis reagent, and, if desired, a resulting compound of the formula V wherein n is zero is converted into a compound of the formula V wherein n is 2, and/or a resulting compound is separated into the individual isomers.

2. Process for the production of compounds of the formula V wherein $R_a$, A and n have the meanings given in claim 1, characterised in that an acyloxyazetidinone of the formula IV wherein Ac is an acyl radical is reacted with a hydroxy-lower-alkyl mercaptan of the formula HS—A—OH in the presence of a suitable base, and, if desired, in a resulting compound the group —S—A—OH is converted into a different —S—A—OH group and/or a resulting compound of the formula II wherein A has the meaning given under the formula V in claim 1 is reacted with a carbonyl compound of the formula III wherein $R_b$ and $R_c$ have the meanings given under the formula III in claim 1, or with a derivative thereof, in the presence of an acid reagent, and in a resulting compound of the formula I wherein R is hydrogen, A has the meaning given under the formula V in claim 1 and $R_b$ and $R_c$ have the meanings given under the formula III in claim 1 and n is zero, a radical $R_a$ is introduced, and, if desired, a resulting compound is converted into a different compound of the formula I wherein n is 2, and/or an isomeric mixture obtainable according to the process is separated into the individual isomers, and a compound of the formula I is solvolysed in the presence of

a suitable solvolysis reagent, and, if desired, a resulting compound of the formula V wherein n is zero is converted into a compound of the formula V wherein n is 2, and/or a compound obtainable according to the process is separated into the individual isomers.

3. Process according to claim 1 for the production of trans-3-(1-p-nitrobenzyloxycarbonyloxyethyl)-4-(2-hydroxyethylsulfonyl)-2-oxo-azetidine of the formula V, characterised in that 4-acetoxyazetidin-2-one is reacted with 2-mercaptoethanol in the presence of a suitable base, the resulting 4-(2-hydroxyethylthio)-2-oxo-azetidine is reacted with acetonedimethyl ketal in the presence of an acid reagent, in the resulting 2,2-dimethyl-9-oxo-3-oxa-6-thia-1-azabicyclo[$5.2.0^{1,7}$]nonane the 1-hydroxyethyl radical is introduced, the resulting 2,2-dimethyl-trans-8-(1-hydroxyethyl)-9-oxo-3-oxa-6-thia-1-azabicyclo[$5.2.0^{1,7}$]-nonane is converted into 2,2-dimethyl-trans-8-(1-p-nitrobenzyloxycarbonyloxyethyl)-9-oxo-3-oxa-6-thia-1-azabicyclo[$5.2.0^{1,7}$]nonane-6-dioxide, and this compound is solvolysed in the presence of a suitable solvolysis reagent.

4. Process according to claim 3 for the production of trans-3-(1-p-nitrobenzyloxycarbonyloxyethyl)-4-(2-hydroxyethylsulfonyl)-2-oxo-azetidine of the formula V, characterised in that 4-acetoxyazetidin-2-one is reacted with 2-mercaptoethanol in the presence of an aqueous sodium hydroxide solution, the resulting 4-(2-hydroxyethylthio)-2-oxo-azetidine is reacted with acetonedimethyl ketal in the presence of boron trifluoride etherate, in the resulting 2,2-dimethyl-9-oxo-3-oxa-6-thia-1-azabicyclo[$5.2.0^{1,7}$]-nonane the hydroxyethyl radical is introduced, and the resulting 2,2-dimethyl-trans-8-(1-hydroxyethyl)-9-oxo-3-oxa-6-thia-1-azabicyclo[$5.2.0^{1,7}$]nonane is converted into 2,2-dimethyl-trans-8-(p-nitrobenzyloxycarbonyloxyethyl)-9-oxo-6-thia-1-azabicyclo[$5.2.0^{1,7}$]nonane-6-dioxide, and this compound is solvolysed in the presence of glacial acetic acid.

5. Process for the production of compounds of the formula I wherein $R_a$ and A have the meanings given under the formula V in claim 1, and $R_b$ and $R_c$ have the meanings given under the formula III in claim 1, and n is zero, characterised in that in a compound of the formula I wherein R represents hydrogen, A has the meaning given under the formula V in claim 1, and $R_b$ and $R_c$ have the meanings given under the formula III in claim 1, and n is zero, a radical $R_a$ is introduced.

6. Compounds of the formula V according to claim 1, wherein $R_a$, A and n have the meanings given in claim 1, the stereoisomers thereof and mixtures of these stereoisomers.

7. Compounds of the formula V according to claim 1, wherein $R_a$, A and n have the meanings given in claim 1.

8. Compounds according to claim 6 of the formula V wherein $R_a$ represents lower alkyl, lower alkenyl, hydroxy-lower-alkyl, lower-alkoxy-lower-alkyl, lower-alkanoyloxy-lower-alkyl, aryl-lower-alkoxycarbonyloxy-lower-alkyl unsubstituted or substituted by lower alkyl, lower alkoxy, halogen and/or nitro, or is hydroxysulfonyloxy-lower-alkyl present in salt form, A is ethylene or 1,2-propylene, and n is zero or 2, the stereoisomers of compounds of the formula V and mixtures of these stereoisomers.

9. Compounds according to claim 7 of the formula V wherein $R_a$ has the meanings given in claim 8, A is ethylene, and n is zero or 2.

10. Compounds according to claim 8 of the formula V wherein $R_a$ is lower alkyl having up to 4 carbon atoms, hydroxy-lower-alkyl having up to 4 carbon atoms, lower alkenyl having up to 4 carbon atoms, lower-alkoxy-lower-alkyl, wherein lower alkyl and lower alkoxy each contain up to 4 carbon atoms, lower-alkanoyloxy-lower-alkyl, wherein lower alkanoyloxy and lower alkyl each contain up to 4 carbon atoms, phenyl-lower-alkoxycarbonyloxy-lower-alkyl unsubstituted or substituted by halogen and/or nitro, or hydroxysulfonyloxy-lower-alkyl present in salt form, wherein lower alkyl contains up to 4 carbon atoms, A is ethylene or 1,2-propylene, and n is zero or 2, the stereoisomers of compounds of the formula I and mixtures of these stereoisomers.

11. Compounds according to claim 9 of the formula V wherein $R_a$ has the meanings given in claim 10, A represents ethylene, and n is zero or 2.

12. Compounds according to claim 10 of the formula V, wherein $R_a$ is lower alkyl having up to 4 carbon atoms, lower alkenyl having up to 4 carbon atoms, 1-hydroxy-lower-alkyl having up to 4 carbon atoms, or phenyl-lower-alkoxycarbonyloxy-lower-alkyl unsubstituted or substituted by halogen and/or nitro, A represents ethylene of 1,2-propylene, and n is zero or 2, the stereoisomers of compounds of the formula I and mixtures of these stereoisomers.

13. Compounds according to claim 11 of the formula V, wherein $R_a$ has the meanings given in claim 12, A represents ethylene, and n is zero or 2.

14. Trans-3-(1-p-nitrobenzyloxycarbonyloxyethyl)-4-(2-hydroxyethylsulfonyl)-2-oxo-azetidine.

15. Compounds of the formula I according to claim 1, wherein R represents hydrogen or $R_a$, and wherein $R_a$, A and n have the meanings given under the formula V in claim 1, annd $R_b$ and $R_c$ have the meanings given under the formula III in claim 1, the stereoisomers thereof and mixtures of these stereoisomers.

16. Compounds of the formula I according to claim 1, wherein R represents hydrogen or $R_a$, and wherein $R_a$, A and n have the meanings given under the formula V in claim 1, and $R_b$ and $R_c$ have the meanings given under the formula III in claim 1.

17. Compounds according to claim 15 of the formula I wherein R represents hydrogen or $R_a$, and wherein $R_a$, A and n have the meanings given in claim 8, and each of the radicals $R_b$ and $R_c$ represent hydrogen, lower alkyl, phenyl-lower-alkyl or phenyl, or, when taken together, they represent lower

alkylene having 3 to 6 carbon atoms, the stereoisomers thereof and mixtures of these stereoisomers.

18. Compounds according to claim 16 of the formula I wherein R represents hydrogen or $R_a$, and wherein $R_a$ has the meanings given in claim 8, and A represents ethylene, and n is or 2, and $R_b$ and $R_c$ have the meanings given in claim 17.

19. Compounds according to claim 17 of the formula I wherein R is hydrogen or $R_a$, and wherein $R_a$, A and n have the meanings given in claim 10, and each of the radicals $R_b$ and $R_c$ represents hydrogen or lower alkyl having up to 4 carbon atoms, or, when taken together, they represent lower alkylene having 3-6 carbon atoms, the stereoisomers thereof and mixtures of these stereoisomers.

20. Compounds according to claim 18 of the formula I wherein R is hydrogen or $R_a$, and wherein $R_a$ has the meanings given in claim 10, and A is ethylene, and n is zero or 2, and $R_b$ and $R_c$ have the meanings given in claim 19.

21. Compounds according to claim 19 of the formula I wherein R is hydrogen or $R_a$, and wherein $R_a$, A and n have the meanings given in claim 12, each of the radicals $R_b$ and $R_c$ represent lower alkyl, or, when taken together, they represent lower alkylene having 3 to 6 carbon atoms, the stereoisomers thereof and mixtures of these stereoisomers.

22. Compounds according to claim 20 of the formula I wherein R represents hydrogen or $R_a$, and wherein $R_a$ has the meanings given in claim 12, A represents ethylene, and n is zero or 2, and $R_b$ and $R_c$ have the meanings given in claim 21.

23. 2,2-Dimethyl-9-oxo-3-oxa-6-thia-1-azabicyclo$[5.2.0^{1.7}]$nonane.

24. (7R,5R)-2,2,5-Trimethyl-9-oxo-3-oxa-6-thia-1-azabicyclo$[5.2.0^{1.7}]$nonane.

25. (7S,5R)-2,2,5-Trimethyl-9-oxo-3-oxa-6-thia-1-azabicyclo$[5.2.0^{1.7}]$nonane.

26. 2,2-Dimethyl-trans-8-(1-hydroxyethyl)-9-oxo-3-oxa-6-thia-1-azabicyclo$[5.2.0^{1.7}]$nonane.

27. 2,2-Dimethyl-trans-8-allyl-9-oxo-3-oxa-6-thia-1-azabicyclo$[5.2.0^{1.7}]$nonane.

28. 2,2-Dimethyl-trans-8-(1-p-nitrobenzoxycarbonyloxyethyl)-9-oxo-3-oxa-6-thia-1-azabicyclo $[5.2.0^{1.7}]$nonane.

29. 2,2-Dimethyl-trans-8-(1-p-nitrobenzoxycarbonyloxyethyl)-9-oxo-3-oxa-6-thia-1-azabicyclo $[5.2.0^{1.7}]$nonane-6-dioxide.

30. Compounds of the formula

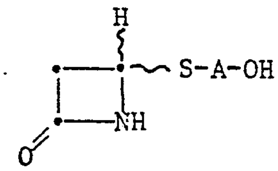

(II)

wherein A represents a lower alkyl radical having 2-3 carbon atoms, the stereoisomers of compounds of the formula II and mixtures of these stereoisomers.

31. Compounds of the formula II wherein A represents a lower alkylene radical having 2-3 carbon atoms.

32. 4-(2-Hydroxyethylthio)-2-oxo-azetidine.

33. (4R)-4-((2R)-1-Hydroxyprop-2-yl-thio)-2-azetidinone.

34. (4S)-4-((2R)-1-Hydroxyprop-2-yl-thio)-2-azetidinone.

35. The compounds obtainable by the process according to claim 1.

36. The compounds obtainable by the process according to claim 5.

**Revendications**

1. Procédé de préparation de composés de formule

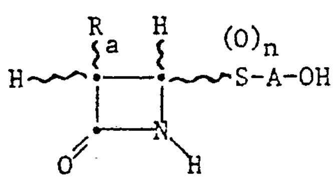

(V)

où $R_a$ représente un radical hydrocarboné aliphatique, cycloaliphatique, cycloaliphatique-aliphatique, aromatique ou araliphatique éventuellement substitué, saturé ou non saturé, lié par l'intermédiaire d'un atome de carbone avec l'atome de carbone du noyau, ayant jusqu'à 18, de préférence jusqu'à 10, atomes de carbone ou un radical hétérocyclyl- ou hétérocyclyl-alcoyle inférieur éventuellement substitué ayant jusqu'à 10 atomes de carbone et de 1 à 4 hétéroatomes du groupe de l'azote, de l'oxygène et/ou du soufre, et où les substituants de ces radicaux $R_a$ sont un hydroxy, un alcoxy inférieur, un alcanoyloxy inférieur, un hydroxysulfonyloxy présent sous forme de sel, un halogène, un mercapto, un alcoyle

inférieur-mercapto, un carboxyle, un alcoxy-inférieur-carbonyle, un carbamoyle, un cyano, un nitro, un sulfo présent sous forme de sel, ou un amino éventuellement mono- ou disubstitué par un alcoyle inférieur ou un acyle ou disubstitué par un alcoyle inférieur, A représente un radical alcoylène inférieur ayant de 2 à 3 atomes de carbone entre le soufre et l'oxygène et n vaut 0 ou 2, de leurs stéréoisomères et des mélanges de ces stéréoisomères, caractérisé en ce qu'on fait réagir une acyloxyazétidinone de formule

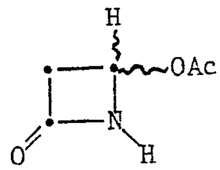

(IV)

où Ac représente un radical acyle, avec un hydroxyalcoyle inférieur-mercaptan de formule HS—A—OH en présence d'une base appropriée et si on le désire en ce qu'on transforme dans un composé obtenu le groupe —S—A—OH en un autre groupe —S—A—OH et en ce qu'on fait réagir un composé obtenu de formule

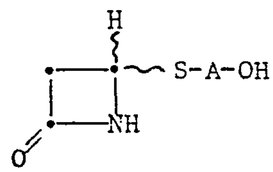

(II)

où A a la signification donnée pour la formule V, avec un composé carbonyle de formule

$$R_b\text{—}(C{=}O)\text{—}R_c \quad \text{(III)}$$

où chacun des radicaux $R_b$ et $R_c$ représente un hydrogène ou un radical organique qui est lié avec un atome de carbone au groupe carbonyle, où $R_b$ et $R_c$ peuvent être liés l'un à l'autre, ou un de ses dérivés, en présence d'un réactif acide, et en ce que dans un composé de formule

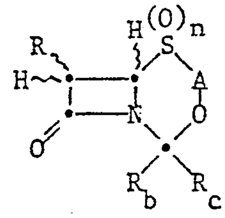

(I)

où R représente un hydrogène, A a la signification donnée pour la formule V, $R_b$ et $R_c$ ont les significations données pour la formule III et n vaut 0, on introduit un radical $R_a$ et si on le désire on transforme un composé obtenu en un composé de formule I où n vaut 2 et/ou on sépare un mélange d'isomères obtenu selon le procédé en les isomères isolés et on solvolyse le composé de formule I en présence d'un réactif de solvolyse approprié et si on le désire on transforme un composé de formule V obtenu où n vaut 0 en un composé de formule V où n vaut 2, et/ou on sépare un composé obtenu en les isomères isolés.

2. Procédé de préparation de composés de formule V, où $R_a$, A et n ont les significations données dans la revendication 1, caractérisé en ce qu'on fait réagir une acyloxyazétidinone de formule IV, où Ac représente un radical acyle, avec un hydroxy-alcoyle inférieur-mercaptan de formule HS—A—OH en présence d'une base appropriée et si on le désire en ce qu'on transforme dans un composé obtenu le groupe —S—A—OH en un autre groupe —S—A—OH et en ce qu'on fait réagir un composé obtenu de formule II, où A a la signification donnée dans la revendication 1 pour la formule V, avec un composé carbonyle de formule III où $R_b$ et $R_c$ ont les significations données dans la revendication 1 pour la formule III, ou un de ses dérivés en présence d'un réactif acide et en ce que dans un composé obtenu de formule I où R représente un hydrogène, A a la signification donnée dans la revendication 1 pour la formule V et $R_b$ et $R_c$ ont les significations données dans la revendication 1 pour la formule III et n vaut 0, on introduit un radical $R_a$ et si on le désire on transforme un composé obtenu en un composé de formule I où n vaut 2, et/ou en ce qu'on dédouble un mélange d'isomères obtenu selon le procédé en les isomères isolés et en ce qu'on solvolyse le composé de formule I en présence d'un réactif de solvolyse approprié et si on le désire en ce qu'on transforme un composé de formule V obtenu, où n vaut 0 en un composé de formule V

où n vaut 2 et/ou en ce qu'on dédouble un composé obtenu selon le procédé en les isomères isolés.

3. Procédé selon la revendication 1 de préparation de trans-3-(1-p-nitrobenzyloxycarbonyloxyéthyl)-4-(2-hydroxyéthylsulfonyl)-2-oxo-azétidine de formule V, caractérisé en ce qu'on fait réagir de la 4-acétoxyazétidin-2-one avec du 2-mercaptoéthanol en présence d'une base appropriée, en ce qu'on fait réagir la 4-(2-hydroxyéthylthio)-2-oxo-azétidine obtenue avec de l'acétone-diméthylcétal en présence d'un réactif acide, en ce que dans le 2,2-diméthyl-9-oxo-3-oxa-6-thia-1-azabicyclo[5.2.$0^{1.7}$]nonane obtenu on introduit le radical 1-hydroxyéthyle, en ce qu'on transforme le 2,2-diméthyl-trans-8-(1-hydroxyéthyl)-9-oxo-3-oxa-6-thia-1-azabicyclo[5.2.$0^{1.7}$]nonane obtenu en le 2,2-diméthyl-trans-8-(1-p-nitrobenzyloxycarbonyloxyéthyl)-9-oxo-3-oxa-6-thia-1-azabicyclo[5.2.$0^{1.7}$]nonane-6-dioxyde et en ce qu'on solvolyse ce composé en présence d'un réactif de solvolyse approprié.

4. Procédé selon la revendication 3 de préparation de trans-3-(1-p-nitrobenzyloxycarbonyloxyéthyl)-4-(2-hydroxyéthylsulfonyl)-2-oxo-azétidine de formule V, caractérisé en ce qu'on fait réagir de la 4-acétoxy-azétidin-2-one avec du 2-mercaptoéthanol en présence d'une solution aqueuse d'hydroxyde de sodium, en ce qu'on fait réagir la 4-(2-hydroxyéthylthio)-2-oxo-azétidine obtenue avec de l'acétone-diméthylcétal en présence d'éthérate de trifluorure de bore, en ce que dans le 2,2-diméthyl-9-oxo-3-oxa-6-thia-1-azabicyclo[5.2.$0^{1.7}$]nonane obtenu on introduit le radical hydroxyéthyle et on transforme le 2,2-diméthyl-trans-8-(1-hydroxyéthyl)-9-oxo-3-oxa-6-thia-1-azabicyclo[5.2.$0^{1.7}$]nonane obtenu en le 2,2-diméthyl-trans-8-(p-nitrobenzyloxycarbonyloxyéthyl)-9-oxo-3-oxa-6-thia-1-azabicyclo[5.2.$0^{1.7}$]nonane-6-dioxyde et en ce qu'on solvolyse ce composé en présence d'acide acétique glacial.

5. Procédé de préparation de composés de formule I, où $R_a$ et A ont les significations données dans la revendication 1 pour la formule V et $R_b$ et $R_c$ ont les significations mentionnées pour la formule III et n vaut 0, caractérisé en ce que dans un composé de formule I où R représente un hydrogène, A a la signification donnée dans la revendication 1 pour la formule V et $R_b$ et $R_c$ ont les significations mentionnées dans la revendication 1 pour la formule III et n vaut 0, on introduit un radical $R_a$.

6. Composés de formule V selon la revendication 1, où $R_a$, A et n ont les significations mentionnées dans la revendication 1, leurs stéréoisomères et les mélanges de ces stéréoisomères.

7. Composés de formule V selon la revendication 1, où $R_a$, A et n ont les significations données dans la revendication 1.

8. Composés selon la revendication 6 de formule V, où $R_a$ représente un alcoyle inférieur, un alcényle inférieur, un hydroxy-alcoyle inférieur, un alcoxy inférieur-alcoyle inférieur, un alcanoyloxy inférieur-alcoyle inférieur, un arylalcoxy inférieur-carbonyloxy-alcoyle inférieur éventuellement substitué par un alcoyle inférieur, un alcoxy inférieur, un halogène et/ou un nitro, ou un hydroxysylfonyloxyalcoyle inférieur présent sous forme de sel, A représente un éthylène ou un 1,2-propylène et n vaut zéro ou 2, les stéréoisomères des composés de formule V et les mélanges de ces stéréoisomères.

9. Composés selon la revendication 7 de formule V, où $R_a$ a les significations données dans la revendication 8, A représente un éthylène et n vaut zéro ou 2.

10. Composés selon la revendication 8 de formule I, où $R_a$ représente un alcoyle inférieur ayant jusqu'à 4 atomes de carbone, un hydroxyalcoyle inférieur ayant jusqu'à 4 atomes de carbone, un alcényle inférieur ayant jusqu'à 4 atomes de carbone, un alcoxy inférieur-alcoyle inférieur, où alcoyle inférieur et alcoxy inférieur contiennent chacun jusqu'à 4 atomes de carbone, un alcanoyloxy inférieur-alcoyle inférieur, où alcanoyloxy inférieur et alcoyle inférieur contiennent chacun jusqu'à 4 atomes de carbone, un phénylalcoxy inférieur-carbonyloxyalcoyle inférieur éventuellement substitué par un halogène et/ou un nitro, ou un hydroxysulfonyloxyalcoyle inférieur présent sous forme de sel, où alcoyle inférieur contient jusqu'à 4 atomes de carbone, A représente un éthylène ou un 1,2-propylène, et n vaut zéro ou 2, les stéréoisomères des composés de formule I et les mélanges de ces stéréoisomères.

11. Composés selon la revendication 9 de formule V, où $R_a$ a les significations données dans la revendication 10, A représente un éthylène et n vaut 0 ou 2.

12. Composés selon la revendication 10 de formule V, où $R_a$ représente un alcoyle inférieur ayant jusqu'à 4 atomes de carbone, un alcényle inférieur ayant jusqu'à 4 atomes de carbone, un 1-hydroxyalcoyle inférieur ayant jusqu'à 4 atomes de carbone ou un phénylalcoxy-inférieur-carbonyloxy-alcoyle inférieur éventuellement substitué par un halogène et/ou un nitro, A représente un éthylène ou un 1,2-propylène et n vaut zéro ou 2, les stéréoisomères des composés de formule I et les mélanges de ces stéréoisomères.

13. Composés selon la revendication 11 de formule V, où $R_a$ a les significations données dans la revendication 12, A représente un éthylène et n vaut 0 ou 2.

14. La trans-3-(1-p-nitrobenzyloxycarbonyloxyéthyl)-4-(2-hydroxyéthylsulfonyl)-2-oxo-azétidine.

15. Composés de formule I selon la revendication 1, où R représente un hydrogène ou $R_a$, et où $R_a$, A et n ont les significations données dans la revendication 1 pour la formule V et $R_b$ et $R_c$ ont les significations données dans la revendication 1 pour la formule III, leurs stéréoisomères et les mélanges de ces stéréoisomères.

16. Composés de formule I selon la revendication 1, où R représente un hydrogène ou $R_a$, et où $R_a$, A et n ont les significations données dans la revendication 1 pour la formule V et $R_b$ et $R_c$ ont les significations données dans la revendicatiton 1 pour la formule III.

17. Composés selon la revendication 15 de formule I, où R représente un hydrogène ou $R_a$ et où $R_a$, A et n ont les significations données dans la revendication 8, et chacun des radicaux $R_b$ et $R_c$ représente

un hydrogène, un alcoyle inférieur, un phénylalcoyle inférieur ou un phényle ou, pris ensemble, un alcoylène en $C_3$ à $C_6$, leurs stéréoisomères et les mélanges de ces stéréoisomères.

18. Composés selon la revendication 16 de formule I, où R représente un hydrogène ou $R_a$ et où $R_a$ a les significations données dans la revendication 8 et A représente un éthylène et n vaut 0 ou 2 et $R_b$ et $R_c$ ont les significations données dans la revendication 17.

19. Composés selon la revendication 17 de formule I, où R représente un hydrogène ou $R_a$ et où $R_a$, A et n ont les significations données dans la revendication 10, et chacun des radicaux $R_b$ et $R_c$ représente un hydrogène ou un alcoyle inférieur ayant jusqu'à 4 atomes de carbone ou, pris ensemble, un alcoylène inférieur en $C_3$ à $C_6$, leurs stéréoisomères et les mélanges de ces stéréoisomères.

20. Composés selon la revendication 18 de formule I, où R représente un hydrogène ou $R_a$ et où $R_a$ a les significations données dans la revendication 10 et A représente un éthylène et n vaut zéro ou 2 et $R_b$ et $R_c$ ont les significations données dans la revendication 19.

21. Composés selon la revendication 19 de formule I, où R représente un hydrogène ou $R_a$ et où $R_a$, A et n ont les significations données dans la revendication 12, chacun des radicaux $R_b$ et $R_c$ représente un alcoyle inférieur ou, pris ensemble, un alcoylène inférieur en $C_3$ à $C_6$, leurs stéréoisomères et les mélanges de ces stéréoisomères.

22. Composés selon la revendication 20 de formule I, où R représente un hydrogène ou $R_a$ et où $R_a$ a la signification donnée dans la revendication 12 et A représente un éthylène et n vaut 0 ou 2 et $R_b$ et $R_c$ ont les significations données dans la revendication 21.

23. 2,2-diméthyl-9-oxo-3-oxa-6-thia-1-azabicyclo[5.2.$0^{1,7}$]nonane.

24. (7R,5R)-2,2,5-triméthyl-9-oxo-3-oxa-6-thia-1-azabicyclo[5.2.$0^{1,7}$]nonane.

25. (7S,5R)-2,2,5-triméthyl-9-oxo-3-oxa-6-thia-1-azabicyclo[5.2.$0^{1,7}$]nonane.

26. 2,2-diméthyl-trans-8-(1-hydroxyéthyl)-9-oxo-3-oxa-6-thia-1-azabicyclo[5.2.$0^{1,7}$]nonane.

27. 2,2-diméthyl-trans-8-allyl-9-oxo-3-oxa-6-thia-1-azabicyclo[5.2.$1^{1,7}$]nonane.

28. 2,2-diméthyl-trans-8-(1-p-nitrobenzoxycarbonyloxyéthyl)-9-oxo-3-oxa-6-thia-1-azabicyclo[5.2.$0^{1,7}$]nonane.

29. 2,2-diméthyl-trans-8-(1-p-nitrobenzoxycarbonyloxyéthyl)-9-oxo-3-oxa-6-thia-1-azabicyclo[5.2.$0^{1,7}$]nonane-6-dioxyde.

30. Composés de formule

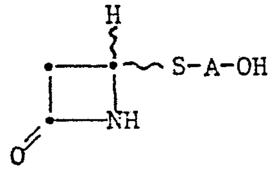

(II)

où A représente un radical alcoylène inférieur en $C_2$ à $C_3$, les stéréoisomères des composés de formule II et les mélanges de ces stéréoisomères.

31. Composés de formule II, où A représente un radical alcoylène inférieur en $C_2$ à $C_3$.

32. 4-(2-hydroxyéthylthio)-2-oxo-azétidine.

33. (4R)-4-((2R)-1-hydroxyprop-2-yl-thio)-2-azétidinone.

34. (4S)-4-((2R)-1-hydroxyprop-2-yl-thio)-2-azétidinone.

35. Les composés que l'on peut obtenir selon le procédé de la revendication 1.

36. Les composés que l'on peut obtenir selon le procédé de la revendication 5.